# EUROPEAN PATENT SPECIFICATION

(11) **EP 1 292 674 B1**
(45) Date of publication and mention of the grant of the patent: **16.07.2008**
(21) Application number: 01934660.0
(22) Date of filing: 04.05.2001
(51) Int. Cl.: C12N 15/12, C12N 15/85, C07K 14/475, A61K 38/18, A61K 48/00, C12Q 1/68

(54) **NUCLEOTIDE SEQUENCES INVOLVED IN INCREASING OR DECREASING MAMMALIAN OVULATION RATE**
NUKLEOTIDSEQUENZEN, DIE AN ERHÖHUNG ODER VERRINGERUNG VON DER OVULATIONSGESCHWINDIGKEIT IN SÄUGERTIEREN BETEILIGT SIND
SEQUENCES NUCLEOTIDIQUES ASSOCIEES A L'AUGMENTATION OU A LA REDUCTION DU TAUX D'OVULATION MAMMALIEN

(30) Priority: 05.05.2000 NZ 50084400
(43) Date of publication of application: 19.03.2003
(73) Proprietor: Biotechvisions Ltd., 00290 Helsinki (FI); AgResearch Limited, 2001 Hamilton (NZ)
(72) Inventor: RITVOS, Olli, Visa-Pekka, 02130 Espoo (FI); DAVIS, George Henry, Invermay Agricultural Centre, Dunedin (NZ); GALLOWAY, Susan May, AgResearch Molecular Bio Unit, P.O. Box 56, Dunedin (NZ); MCNATTY, Kenneth Pattrick, P.O. Box 40063, Upper Hutt (NZ)
(74) Representative: Wilson Gunn
(86) International application number: PCT/NZ2001/000073
(87) International publication number: WO 2001/085926

(56) References cited:
- GALLOWAY SUSAN M ET AL: "Mutations in an oocyte-derived growth factor gene (BMP15) cause increased ovulation rate and infertility in a dosage-sensitive manner." NATURE GENETICS, vol. 25, no. 3, July 2000 (2000-07), pages 279-283, XP002188991 ISSN: 1061-4036
- MONTGOMERY G W ET AL: "PHYSIOLOGY AND MOLECULAR GENETICS OF MUTATIONS THAT INCREASE OVULATION RATE IN SHEEP" ENDOCRINE REVIEWS, vol. 13, no. 2, 1992, pages 309-328, XP001052902 ISSN: 0163-769X
- AALTONEN JOHANNA ET AL: "Human growth differentiation factor 9 (GDF-9) and its novel homolog GDF-9B are expressed in oocytes during early folliculogenesis." JOURNAL OF CLINICAL ENDOCRINOLOGY & METABOLISM, vol. 84, no. 8, August 1999 (1999-08), pages 2744-2750, XP001052895 ISSN: 0021-972X
- MONTGOMERY GRANT W ET AL: "Genes controlling ovulation rate in sheep." REPRODUCTION (CAMBRIDGE), vol. 121, no. 6, June 2001 (2001-06), pages 843-852, XP001052962 ISSN: 1470-1626

## Description

The present invention relates to nucleotide sequences which are involved in increasing or decreasing mammalian ovulation rate.

In particular, the invention broadly concerns novel mutations in a gene which is involved in increasing the ovulation rate in heterozygous female mammals; these mutations cause sterility in homozygous female mammals. Knowledge of the mutated gene sequence can be applied to a test for identifying heterozygous or homozygous female and male mammals carrying the mutated gene. This knowledge of the biological function of the gene and its mutations can also be utilised to increase or decrease the ovulation rate of female mammals, or to induce sterility or reduced fertility in female mammals.

### BACKGROUND OF THE INVENTION

No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

The Inverdale high fecundity gene (FecX^{I}) is a major gene for prolificacy in sheep, which was first identified in a Romney flock (Inverdale) consisting of descendants of Romney ewe (A281) all of which had consistently high litter sizes. Segregation studies showed that the gene is carried on the X-chromosome (Davis *et al.,* 1991). A single copy of the gene in heterozygous I+ ewes increases ovulation rate by about one extra egg, and litter size by about 0.6 lambs per ewe lambing. However, homozygous II ewes carrying two copies of the gene have small, non-functional ovaries, and are infertile (Davis *et al*., 1992). Studies on foetal I+ and II sheep demonstrated that ovarian development is normal until approximately day 100 of foetal life: germ cell development, ovarian follicular formation and the earliest stages of follicular growth are normal. However, in II foetuses, after day 100 of foetal life follicular development beyond the primary stage of growth is impaired, and normal secondary follicles are not observed (Smith *et al*., 1997). As oocytes in II animals increase in diameter (>40 µm) there is no evidence of granulosa cell proliferation, in contrast to what would normally be observed (Braw-Tal *et al*., 1993; McNatty *et al*., 1995a; Smith *et al*., 1997). Thus the presence of infantile, non-functional ovaries in foetal, neonatal and adult II animals is due to a block in follicular development beyond the primary stage of growth.

A second prolific Romney flock (Hanna, 1995), with no known connection to the Inverdale flock, was also shown to carry an X-linked mutation with a similar phenotype to Inverdale. Confirmation that the Hanna animals carried a mutation (FecX^{H}) in the Inverdale gene was obtained when homozygous infertile females were produced by mating Inverdale carrier rams with carrier Hanna ewes (Davis *et al.,* 1995). This Hanna line was maintained at Invermay as a distinct group alongside the original Inverdale line.

As part of the search for the gene responsible for the Inverdale trait, the inventors have constructed a genetic linkage map of the sheep X-chromosome (Galloway *et al*., 1996), and localised the Inverdale gene to a 10 cM region flanked by microsatellite markers (Galloway *et al*., 1999). Localisation of the gene to the sheep X-chromosome narrows the search for candidates to those genes which map to other mammalian X-chromosomes, because, almost without exception, genes on the X-chromosome of one mammal are also present on the X-chromosomes of other mammalian species (Ohno, 1973).

Inheritance of the Inverdale gene on the X-chromosome provides a convenient means of producing prolific single copy Inverdale carrier ewes, because all daughters of an Inverdale carrier ram will inherit the gene. The breeder of the rams uses a genetic marker test to identify carrier rams for sale, and commercial breeders purchase these rams to generate prolific ewes, which are subsequently mated to a terminal sire to produce progeny for slaughter. Commercial use of the Inverdale gene has been shown to be highly beneficial in an existing terminal sire mating system, with an added value over a normal ram of $1760 per Inverdale ram purchased (Amer *et al*., 1998). Production of elite rams carrying the gene requires the ability to distinguish between non-carriers (++ females or +Y males) and single copy carriers (I+ females or IY males).

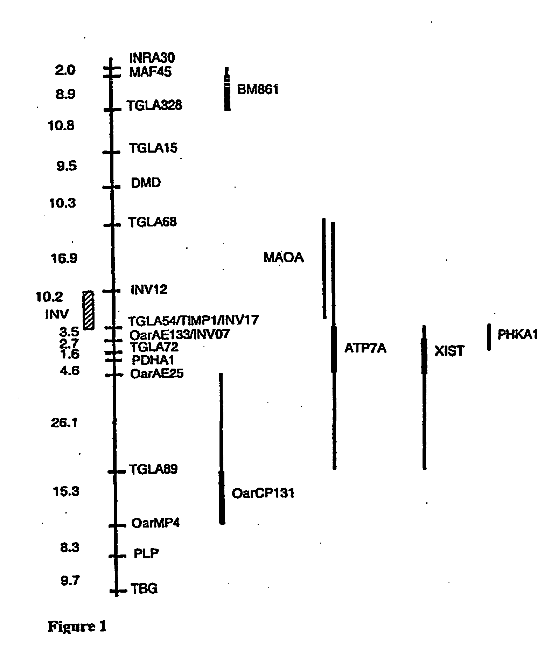

A genetic marker test was developed on the basis of inheritance of flanking microsatellite markers around the gene (i.e. a haplotype test) (Galloway *et al*., 1999); this is illustrated in Figure 1. However, the current test can only identify those animals which have inherited the Inverdale haplotype from a known carrier, and is not 100% accurate, because it does not detect the Inverdale gene itself. The haplotype from the same region of the X-chromosome in sheep of the Hanna pedigree, which carry the unrelated version of Inverdale, was different from the haplotype seen in descendants of A281.

In 1996 growth differentiation factor 9 (GDF-9), a member of the transforming growth factor beta (TGF-β) superfamily, was shown to be specifically expressed in the oocyte of adult mice, where it is required for folliculogenesis (Dong *et al*., 1996). GDF-9 messenger RNA is synthesised only in the oocyte, from the primordial/primary one-layer follicular stage until after ovulation, and female GDF-9 knockout mice are infertile due to a block in follicular development at this primary one-layer follicle stage. Animals homozygous for the Inverdale gene are infertile, with a similar phenotype to the GDF-9 knockout mouse (McNatty *et al*., 1995b). GDF-9 was subsequently mapped to sheep chromosome 5, and therefore could not be responsible for the Inverdale phenotype (Sadighi *et al*., 1998).

A second related member of this family, GDF-9B, also called BMP 15, was identified in mouse and human ovaries, and found to be co-expressed with GDF-9 (Laitinen *et al.,* 1998, Dube *et al*., 1998). BMP15 was mapped to the X-chromosome in mice, close to Fscl (Dube *et al*., 1998). Fscl (fibrous sheath component) is also known as Akap4 (A kinase anchor protein 4), which has been mapped to the mouse X-chromosome at 1.6 cM from the centromere (Mouse Genome Database (MGD), October 1999) and to band p11.2 of the human X-chromosome (Dube *et al.,* 1998). Preliminary studies in Inverdale sheep (++, I+ and II genotypes), using a molecular probe that does not distinguish between the genotypes, show that GDF-9B mRNA is expressed in oocytes of primary but not primordial follicles, and that expression of this mRNA within the ovary is exclusive to oocytes (Galloway *et al* 2000.

Members of the TGF-β superfamily have similar gene structures. The GDF-9B coding region is contained within two exons separated by an intron of 4.2 kb (human) and 3.5 kb (mouse) (Dube *et al*., 1998). In humans the full-length 1176 bp coding sequence produces a 392 amino acid prepropeptide, the first 17 amino acids of which correspond to a secretory signal. The full-length prepropeptide in human and mouse includes the processing site for proteolytic cleavage to release a 125 amino acid mature active C-terminal peptide and an N-terminal propeptide product (Laitinen *et al*., 1998, Dube *et al*., 1998). The intron sequence lies within the propeptide domain, so that the entire mature coding region is found within exon 2.

The sequence of the human BMP15 (GDF-9B) wild type gene is disclosed in US 5,728,679 and US 5,635,372. The wild type protein is disclosed as being useful in the treatment of bone and cartilage and/or other connective tissue defects, and in wound healing and tissue repair.

The inventors have now identified a mutated form of the sheep GDF-9B gene in sheep expressing the Inverdale or Hanna phenotype, and discovered for the first time that this mutated form of GDF-9B is responsible for the enhanced ovulation seen in these sheep and for the sterility seen in homozygous sheep.

The present invention is broadly directed to the mutated sequence and its corresponding encoded protein.

### SUMMARY OF THE INVENTION

Accordingly, in one aspect, the present invention provides an isolated mutated GDF-9B nucleic acid molecule encoding a polypeptide capable of modulating the ovulation rate of a female mammal, comprising a nucleotide sequence selected from the group consisting of:
a) SEQ ID NO: 1, SEQ ID NO: 3, SEQ ID NO: 5, or SEQ ID NO: 7; and
b) a sequence complementary to the molecule(s) defined in (a).

The nucleic acid molecule may be an RNA, cRNA, genomic DNA or cDNA molecule, and may be single- or double-stranded. The nucleic acid molecule may also optionally comprise one or more synthetic, non-natural or altered nucleotide bases, or combinations thereof.

The present invention further provides a method of identifying a mammal which carries a mutated GDF-9B nucleic acid molecule, said method comprising the steps of:
(i) isolating DNA from a mammal tissue or blood sample;
(ii) optionally isolating GDF-9B DNA from the DNA obtained at step (i);
(iii) optionally probing the DNA with a probe complementary to the mutated GDF-9B DNA of the invention;
(iv) optionally amplifying the amount of mutated GDF-9B DNA; and
(v) determining whether the GDF-9B sequence DNA obtained in Step (iv) carries a mutation associated with sterility, or with increased or decreased ovulation.

Preferably the amplication step (iv) may be performed by any convenient method, such as the polymerase chain reaction, or ligase chain reaction.

According to still a further aspect the present invention there is provided a genetic marker for DNA- assisted selection for enhanced ovulation or sterility in a mammal, comprising a nucleic acid molecule which specifically hybridises under stringent conditions to a nucleotide sequence according to the first aspect of the invention, or to genomic DNA encoding the mutated GDF-9B nucleic acid molecule.

The mammal may be a human, or a domestic, companion, zoo or feral mammal. Preferably the mammal is selected from humans, sheep, cattle, goats, deer, horses, camelids, possums, pigs, mice, rats, weasels, rabbits, hares, ferrets, cats and dogs.

In a further aspect, the present invention provides an isolated polypeptide encoded by a nucleic acid molecule having a sequence set out in one of (a)-(b) above. Preferably the polypeptide has an amino acid sequence selected from the group consisting of SEQ ID NO: 2, SEQ ID NO: 4 or SEQ ID NO: 6.

In a still further aspect, the invention provides an isolated nucleic acid molecule which encodes a polypeptide substantially as described above.

In a further aspect, the ovulation rate of a female mammal is modulated by an effective amount of a mutated GDF-9B polypeptide.

The ovulation rate of a female mammal which does not carry a mutated GDF-9B nucleic acid molecule may be increased by an effective amount of a mutated GDF- 9B polypeptide or a functional variant or fragment thereof which is capable of modulating the ovulation rate of a female mammal.

In another aspect, the invention provides an agent for increasing or reducing the ovulation rate, or for inducing sterility in a female mammal, consisting of
an immunising-effective amount of a mutated GDF-9B polypeptide.

In yet a further aspect, the invention provides a composition comprising an effective amount of a mutated GDF-9B polypeptide, together with a pharmaceutically or veterinarily acceptable carrier or diluent.

In still a further aspect, the invention provides a composition comprising an effective amount of an agent selected from the group consisting of
a mutated GDF-9B polypeptide according to the invention
together with a pharmaceutically or veterinarily acceptable carrier or diluent.

According to yet a further aspect, the invention provides a construct or vector comprising a nucleic acid molecule substantially as described above.

The present invention also provides a host cell transformed with a vector or construct comprising a nucleic acid molecule of the invention.

According to a further aspect, the invention provides an isolated nucleic acid molecule comprising a nucleotide sequence selected from the group consisting of SEQ ID NO:12 or SEQ ID NO: 14.

The invention also provides an isolated polypeptide comprising an amino acid sequence of SEQ ID NO: 13.

While the invention is broadly as defined above, it will be appreciated by those persons skilled in the art that it is not limited thereto, and that it also includes embodiments of which the following description gives examples.

### BRIEF DESCRIPTION OF DRAWINGS

In particular, preferred aspects of the invention will be described in relation to the accompanying drawings, in which:
Figure 1 shows a genetic linkage map of the ovine X-chromosome. Genetic distances are in Kosamabi centiMorgans (cM). The Inverdale gene maps into the region indicated by a hatched bar.
Figure 2a shows the nucleotide sequence of exon 2 of GDF-9B in Inverdale sheep. The position of the Inverdale T to A nucleotide substitution (92 nucleotides beyond the processing site) is marked in bold. The triplet codon affected by this substitution is underlined. The processing site for proteolytic cleavage of propeptide from the mature fragment and the TGA stop codon are boxed. The mature peptide coding sequence is between the two boxes.
Figure 2b shows the nucleotide sequence of exon 2 of GDF-9B in Hanna sheep. The position of the Hanna C to T nucleotide substitution (67 nucleotides beyond the processing site) is marked in bold. The triplet codon affected by this substitution is underlined. The processing site for proteolytic cleavage of propeptide from mature fragment and the TGA stop codon are boxed. The mature peptide coding sequence is between the two boxes.
Figure 2c shows the nucleotide sequence of bp 394-599 of Figure 2a.
Figure 2d shows the nucleotide sequence of bp 394-599 of Figure 2b.
Figure 2e shows the nucleotide sequence of bp 472-486 of Figure 2a.
Figure 2f shows the nucleotide sequence of bp 448-462 of Figure 2b.
Figure 3a shows the deduced amino acid sequence of the GDF-9B Inverdale protein encoded by the nucleotide sequence of Figure 2a. The mature GDF-9B is shown in normal type and the portion of the propeptide is in italics. The amino acid (Aspartic acid, D) produced by the Inverdale base substitution is marked in bold.
Figure 3b shows the deduced amino acid sequence of the truncated GDF-9B Hanna protein encoded by the nucleotide sequence of Figure 2b. The mature GDF-9B peptide is shown in normal type and the portion of the propeptide is in italics. The wild type amino acid (Glutamine, Q) becomes a stop codon (END) in the Hanna mutant.
Figure 4 shows a comparison of predicted amino acid sequence of sheep GDF-9B with human and mouse. Numbers in brackets above the line indicate amino acid positions of the mature peptide. The open triangle shows the position of the Leu polymorphism, and the black triangle indicates the position of the single intron. The RRAR putative processing site and the conserved cysteins are shaded grey. Positions of the FecX^{I} and FecX^{H} mutations at amino acids 23 and 31 are in bold.
Figure 5 shows a chromatogram of GDF-9B sequence from Inverdale, Hanna and wildtype sheep showing region where mutations occur.
Figure 6 shows the alignment of mutated region of predicted FecXI protein with TGFβ superfamily members from other species.
Figure 7 shows a linkage map of the region of sheep X chromosome containing the GDF9-B gene.
Figure 8 shows the results of a SNP variant detection assay of sheep carrying Inverdale *FecX^{I}* mutation, and non-carriers, using *Xba*I digestion of a forced PCR frag) non-carrier, an( I+) heterozygote and an(II) homoxygote carrier are shown beside heterozygote females (samples A1, A2), carrier rams (samples A5, A10) and non-carrier rams (samples A3, A4, A6, A7, A8, A11, A12 and A13).

### DETAILED DESCRIPTION OF THE INVENTION

The mutations in the GDF-9B gene found in Inverdale and Hanna sheep have been shown for the first time to be responsible for the increased ovulation rates seen in heterozygous animals and for sterility seen in homozygous animals.

For the purposes of the specification it will be clearly understood that the word "comprising" means "including but not limited to," and that the word "comprises" has a corresponding meaning.

The term "isolated" means substantially separated or purified away from contaminating sequences in the cell or organism in which the nucleic acid naturally occurs, and includes nucleic acids purified by standard purification techniques as well as nucleic acids prepared by recombinant technology, including PCR technology, and nucleic acids which have been synthesised. Preferably, the nucleic acid molecule is isolated from the genomic DNA of sheep expressing the Inverdale or Hanna phenotype.

The term "modulation of ovulation" means increasing or decreasing the rate of ovulation compared to the rate observed in an untreated mammal.

The term "ligand" refers to any molecule which can bind to another molecule such as a polypeptide or peptide, and should be taken to include, but not be limited to, antibodies and phage display molecules.

The probe and primers used in this method also form a part of this invention. Said probes and primers may comprise a fragment of the nucleic acid molecule of the invention capable of hybridising under stringent conditions to a mutated GDF-9B gene sequence. Such probes and primers are also useful, in studying the structure and function of the mutated gene and for obtaining homologs of the gene from mammals other than sheep expressing the Inverdale or Hanna phenotype.

Nucleic acid probes and primers can be prepared based on nucleic acids according to the present invention. A "probe" comprises an isolated nucleic acid attached to a detectable label or reporter molecule. Typical labels include radioactive isotopes, ligands, chemiluminescent or fluorescent agents, and enzymes.

A "fragment" of a nucleic acid is a portion of the nucleic acid that is less than full length, and comprises at least a minimum sequence capable of hybridising specifically with a nucleic acid molecule according to the invention, or a sequence complementary thereto, under stringent conditions as defined below. A "fragment" of a polypeptide is a portion of the polypeptide which is less than full length, but which still retains the biological function of either increasing or decreasing the ovulation rate of a mammal, or causing sterility in a mammal. Hence, a fragment according to the invention has at least one of the biological activities of the nucleic acid or polypeptide of the invention.

"Primers" are short nucleic acids, preferably DNA oligonucleotides 15 nucleotides or more in length, which are annealed to a complementary target DNA strand by nucleic acid hybridisation to form a hybrid between the primer and the target DNA strand, then extended along the target DNA strand by a polymerase, preferably a DNA polymerase. Primer pairs can be used for amplification of a nucleic acid sequence, e.g. by the polymerase chain reaction (PCR) or other nucleic acid amplification methods well known in the art. PCR-primer pairs can be derived from the sequence of a nucleic acid according to the present invention, for example, by using computer programs intended for that purpose such as Primer (Version 0.5^{©} 1991, Whitehead Institute for Biomedical Research, Cambridge, MA).

Methods for preparing and using probes and primers are described, for example, in Sambrook et al. Molecular Cloning: A Laboratory Manual, 2nd ed, vol. 1-3, ed Sambrook et al. Cold Spring Harbour Laboratory Press, Cold Spring Harbour, NY,1989.

Probes or primers can be free in solution or covalently or noncovalently attached to a solid support by standard means.

"Stringent conditions" for the amplification of a target nucleic acid sequence (eg by PCR) using a particular amplification primer pair, are conditions that permit the primer pair to hybridise only to the target nucleic acid sequence to which a primer having the corresponding wild type sequence (or its complement) would bind.

Nucleic acid hybridisation is affected by such conditions as salt concentration, temperature, or organic solvents, in addition to the base composition, length of the complementary strands, and the number of nucleotide base mismatches between the hybridising nucleic acids, as will be readily appreciated by those skilled in the art.

When referring to a probe or primer, the term "specific for (a target sequence)" indicates that the probe or primer hybridises under stringent conditions only to the target sequence in a given sample comprising the target sequence.

In one embodiment, the invention provides a genetic marker for DNA-assisted selection of animals for increased ovulation or sterility in sheep, goats, cattle, deer, mice, rats or any other commercially important mammal. The invention provides a means of using a nucleic acid molecule containing sequence derived from the mutated GDF-9B DNA sequence, to identify sequence variants in individual animals that are associated with increased ovulation or sterility of that animal. Although these variants may not necessarily give rise to the increased ovulation or sterility trait directly, they will be closely enough associated with it to predict the trait. The methods by which these sequence variants are identified are known in the art, and include, but are not limited to, restriction fragment length polymorphism (RFLP), AFLP, direct sequencing of DNA within or associated with the mutated GDF-9B gene, or identification and characterisation of variable number of tandem repeats (VNTR), or microsatellite polymorphisms (di-or tri-nucleotide repeats), detection and characterisation of single nucleotide polymorphisms (SNP's).

The polypeptide may be produced by expression of a suitable vector comprising the nucleic acid molecule of the invention or a functional variant or fragment thereof, in a suitable host cell as would be understood by a person skilled in the art.

The cloning vector may be selected according to the host or host cell to be used. Useful vectors will generally have the following characteristics:
(a) the ability to self-replicate;
(b) the possession of a single target for any particular restriction endonuclease; and
(c) desirably, carry genes for a readily selectable marker such as antibiotic resistance.

Two major types of vector possessing these characteristics are plasmids and bacterial viruses (bacteriophages or phages). Presently preferred vectors may include the following: the pUC, pBlueScript, pGEM, PGEX, pBK-CMV, lambda ZAP, lambda GEM and pSP series. However, this list should not be seen as limiting the scope of the present invention.

The DNA molecules of the invention may be expressed by placing them in operable linkage with suitable control sequences in a replicable expression vector. Control sequences may include origins of replication, a promoter, enhancer and transcriptional terminator sequences amongst others. The selection of the control sequence to be included in the expression vector is dependent on the type of host or host cell intended to be used for expressing the DNA.

Generally, procaryotic, yeast or mammalian cells are useful hosts. Also included within the term hosts are plasmid vectors. Suitable procaryotic hosts include E. coli, Bacillus species and various species of Pseudomonas. Commonly used promoters such as β-lactamase (penicillinase) and lactose (lac) promoter systems are all well known in the art. Any available promoter system compatible with the host of choice can be used. Vectors used in yeast are also available and well known. A suitable example is the 2 micron origin of replication plasmid.

Similarly, vectors for use in mammalian cells are also well known. Such vectors include well known derivatives of SV-40, adenovirus, retrovirus-derived DNA sequences, Herpes simplex viruses, and vectors derived from a combination of plasmid and phage DNA.

Further eucaryotic expression vectors are known in the art (e.g. P.J. Southern and P.Berg, J. Mol. Appl. Genet. 1 327-341 (1982); S. Subramani et al., Mol.Cell.Biol. 1, 854-864 (1981); R J. Kaufmann and P.A. Sharp, "Amplification and Expression of Sequences Cotransfected with a Modular Dihydrofolate Reducase Complementary DNA Gene, J. Mol. Biol. 159, 601-621 (1982); R J. Kaufmann and P.A. Sharp, Mol.Cell.Biol. 159, 601-664(1982); S.I. Scahill et al., "Expressions And Characterisation Of The Product Of A Human Immune Interferon DNA Gene In Chinese Hamster Ovary Cells," Proc. Natl. Acad. Sci. USA. 80, 4654-4659 (1983); G. Urlaub and L.A. Chasin, Proc. Natl. Acad. Sci. USA. 77, 4216-4220, (1980).

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the lac system, the trp system, the tac system, the trc system, major operator and promoter regions of phage lambda, the glycolytic promoters of yeast acid phosphatase, e.g. Pho5, the promoters of the yeast alpha-mating factors, and promoters derived from polyoma, adenovirus, retrovirus, and simian virus, e.g. the early and late promoters of SV40, and other sequences known to control the expression of genes of prokaryotic and eucaryotic cells and their viruses or combinations thereof.

In the construction of a vector it is also an advantage to be able to distinguish the vector incorporating the foreign DNA from unmodified vectors by a convenient and rapid assay. Reporter systems useful in such assays include reporter genes, and other detectable labels which produce measurable colour changes, antibiotic resistance and the like. In one preferred vector, the β-galactosidase reporter gene is used, which gene is detectable by clones exhibiting a blue phenotype on X-gal plates. This facilitates selection. In one embodiment, the β-galactosidase gene may be replaced by a polyhedrin-encoding gene; which gene is detectable by clones exhibiting a white phenotype when stained with X-gal. This blue-white color selection can serve as a useful marker for detecting recombinant vectors.

Once selected, the vectors may be isolated from the culture using routine procedures such as freeze-thaw extraction followed by purification.

For expression, vectors containing the DNA of the invention and control signals are inserted or transformed into a host or host cell. Some useful expression host cells include well-known prokaryotic and eucaryotic cells. Some suitable prokaryotic hosts include, for example, E.coli, such as E. coli, S G-936, E. coli HB 101, E. coli W3110, E.coli X1776, E. coli, X2282, E. coli, DHT, and E. coli, MR01, Pseudomonas, Bacillus, such as Bacillus subtilis, and Streptomyces. Suitable eucaryotic cells include yeast and other fungi, insect, animal cells, such as COS cells and CHO cells, human cells in tissue culture.

Depending on the host used, transformation is performed according to standard techniques appropriate to such cells. For prokaryotes or other cells that contain substantial cell walls, the calcium treatment process (Cohen, S N Proceedings, National Academy of Science, USA 69 2110 (1972)) may be employed. For mammalian cells without such cell walls the calcium phosphate precipitation method of Graeme and Van Der Eb, Virology 52:546 (1978) is preferred. Transformations in yeast according to the method of Van Solingen et al. J.Bact. 130: 946 (1977) and Hsiao et al. Proceedings, National Academy of Science, 76: 3829 (1979).

Upon transformation of the selected host with an appropriate vector the polypeptide or peptide encoded can be produced, often in the form of fusion protein, by culturing the host cells. The polypeptide or peptide of the invention may be detected by rapid assays as indicated above. The polypeptide or peptide is then recovered and purified as necessary. Recovery and purification can be achieved using any of those procedures known in the art, for example by absorption onto and elution from an anion exchange resin. This method of producing a polypeptide or peptide of the invention constitutes a further aspect of the present invention.

Host cells transformed with the vectors of the invention also form a further aspect of the invention.

In addition, nucleotides .and peptides having substantial identity to the nucleotide and amino acid sequences of the invention can also be employed in preferred embodiments. Here "substantial identity" means that two sequences, when optimally aligned such as by the programs GAP or BESTFIT (nucleotides and peptides) using default gap weights, or as measured by computer algorithm BLASTP (peptides) or BLAST X (nucleotides), share at least 60%, preferably 75%, and most preferably 90-95% sequence identity.

Preferably residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

The term "variant" as used herein includes nucleic acid molecules and polypeptides and peptides having "substantial identity" to the sequences of the invention. The variant may result from modification of the native nucleotide or amino acid sequence by such modifications as insertion, substitution or deletion of one or more nucleotides or amino acids or it may be a naturally-occurring variant. The term "variant" also includes homologous sequences which hybridise to the sequences of the invention under standard hybridisation conditions defined as 2 x SSC at 65°C, or preferably under stringent hybridisation conditions defined as 6 x SCC at 55°C, provided that the variant is capable modulating the ovulation rate of a female mammal. Where such a variant is desired, the nucleotide sequence of the native DNA is altered appropriately. This alteration can be effected by synthesis of the DNA or by modification of the native DNA, for example, by site-specific or cassette mutagenesis. Preferably, where portions of cDNA or genomic DNA require sequence modifications, site-specific primer directed mutagenesis is employed, using techniques standard in the art.

The term "protein or polypeptide" refers to a protein encoded by the nucleic acid molecule of the invention, including fragments, mutations and homologues having the same biological activity i.e. ovulation modulating activity. The protein or polypeptide of the invention can be isolated from a natural source, produced by the expression of a recombinant nucleic acid molecule, or chemically synthesised.

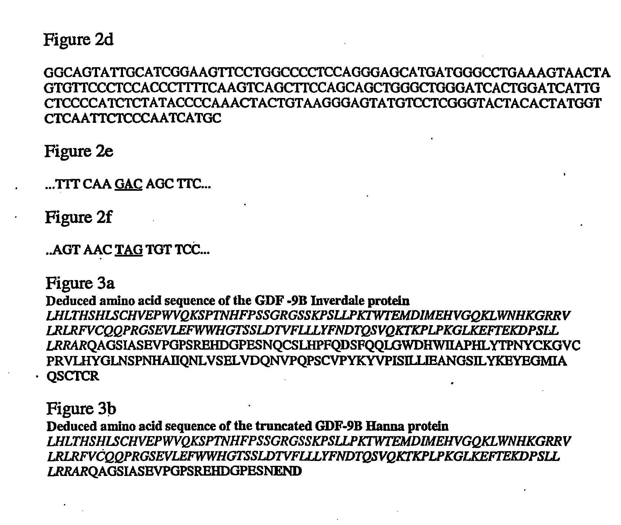

In a further aspect, the invention provides the use of the mutated GDF-9B polypeptide, which has the amino acid sequence set out in figure 3a or 3b in a method of modulating the ovulation rate of a mammal.

The method comprises administering to said mammal an effective amount of mutated GDF-9B.

The modulation of the ovulation rate may comprise inducing sterility in the female mammal by an antibody to mutated GDF-9B to reduce the level of endogenous mutated GDF-9B.

Ligands which bind to polypeptides of the invention may be antibodies. It should be appreciated that the term "antibody" encompasses fragments or analogues of antibodies which retain the ability to bind to a polypeptide of the invention, including but not limited to Fv, F(ab)₂ fragments, ScFv molecules and the like. The antibody may be polyclonal or monoclonal, but is preferably monoclonal.

According to a further aspect, there is provided a composition comprising at least the polypeptide of the invention and a pharmaceutically or veterinarily acceptable carrier or diluent. More than one polypeptide of the invention can of course, be included in the composition.

According to a still further aspect of the present invention there is provided a kit for identifying male and female mammals which carry a single (heterozygous) copy and/or females carrying two (homozygous) copies of a mutated GDF-9B nucleic acid molecule of the invention, comprising:
- primer pairs for amplification of the appropriate region of GDF-9B; and optionally one or more of
- buffer salt solution for the amplification, such as PCR amplification;
- deoxynucleotide mixtures;
- thermostable DNA polymerase enzyme;
- control DNA from the species being tested;
- appropriate standards;
- an appropriate detection system, which could comprise one of the primers in each pair being labelled fluorescently or otherwise, a labelled probe for detection of the product; and
- instructions and protocols for the amplification, and subsequent detection of the amplification products and interpretation of results.

The invention also provides a kit for detecting circulating mutated GDF-9B protein in a mammal. Such a kit may comprise a standard ELISA or enzyme immunoassay format kit familiar to those skilled in the art; for example the kit may contain specific antibody directed to the mutated GDF-9B protein, and standard secondary antibody amplification components to enhance the signal. The antibodies may be conjugated to a fluorescent or radioactive or chemiluminescent label, or the secondary antibody may be labelled. Appropriate solutions, controls, buffers, instructions and protocols may also be supplied.

The invention will now be described in detail by way of reference only to the following non-limiting examples and drawings.

### Animals

The animals tested in this study were from AgResearch Inverdale breeding flocks located at the Invermay Agricultural Centre and Woodlands Research Station, and from the commercial flocks of Mr Arnold Gray, Orawia, Southland (Gray and Davis, 1995). All Inverdale carrier animals were descendants of the original Inverdale ewe (A281) in which the Inverdale gene was first detected.

### Phenotypic measurements

Carrier status of ewes was determined by laparoscopy to identify infertile II ewes, or ovulation rate to distinguish I+ carriers from ++ non-carriers.

Carrier status of rams was either assigned on the basis of ovulation rates of their daughters. Following the discovery of infertile ovaries in II ewes, a faster method for progeny testing of rams was employed by mating each ram to seven to ten I+ ewes and carrying out laparoscopy of the daughters at 6 months. Any resulting infertile II offspring confirm the sire as a carrier. The aim was to produce five daughters per ram, as the probability of an IY ram having no daughters with streak ovaries in a sample of five daughters is only 0.031 (Davis *et al*. 1994).

### DNA purification and sequencing

DNA was purified from the white blood cells present in 5 to 10 ml of whole blood from each animal (Montgomery and Sise, 1990). Sequencing of all subclones and PCR products was carried out by the commercial service operated by the University of Otago Centre for Gene Research (ABI 373 automated sequencer).

### DNA markers

Microsatellite (dinucleotide repeat) markers which amplified DNA from sheep were developed within the AgResearch Molecular Biology Unit as previously described (Galloway *et al*., 1996), or were from the cattle and sheep genetic mapping literature. New markers were mapped on to the sheep X-chromosome as previously described (Galloway *et al*., 1996).

### PCR amplification and restriction digests of ovine GDF-9B gene products

Standard conditions for Polymerase Chain Reaction (PCR) amplification of genomic DNA were used. Primers were designed from the human and mouse sequences (Galloway *et al*., 2000), and shown to amplify gene fragments successfully from sheep DNA. PCR products containing the single nucleotide mutations were digested with commercially-available restriction enzymes SpeI, BsrSI and/or XbaI using standard conditions recommended by the manufacturer. PCR products and restriction fragment products were identified by electrophoretic separation in 2 - 3% agarose gels alongside commercially available DNA size markers.

### Sequencing and Mutation Detection Methods

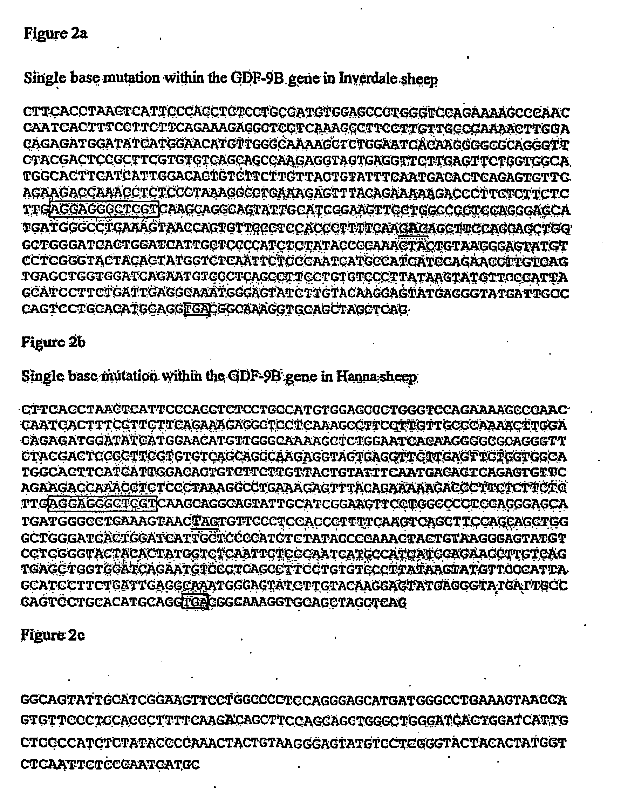

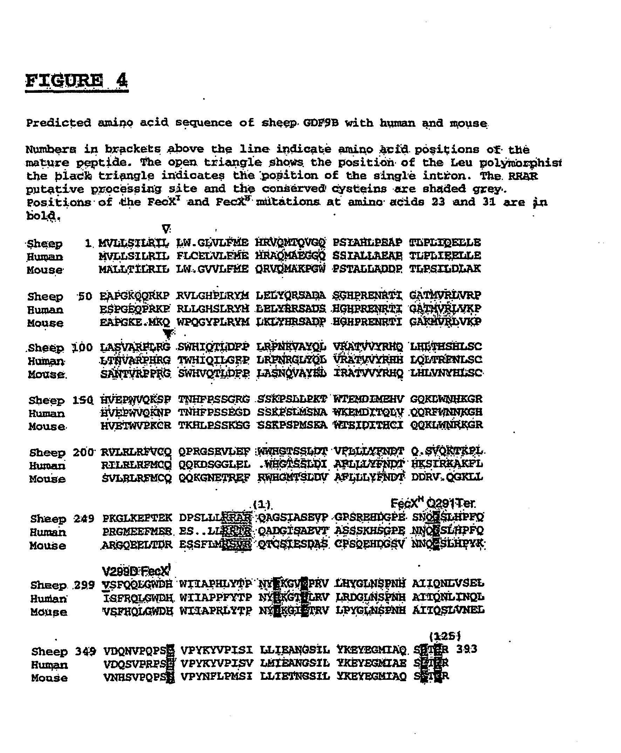

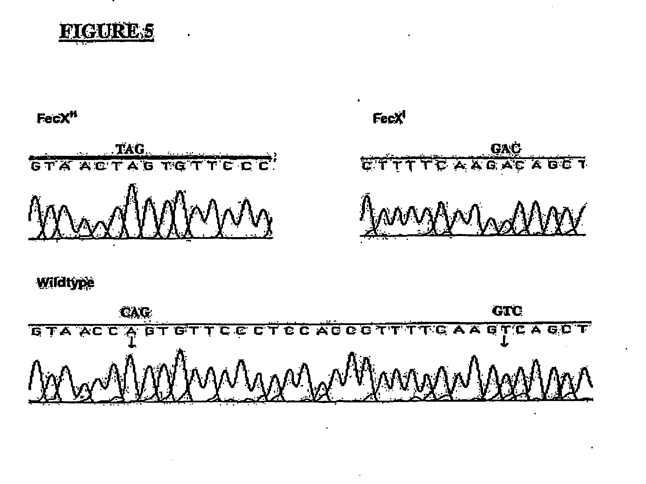

We sequenced the sheep GDF-9B gene in all three genotypes (Inverdale *FecX^{I}*, Hanna *FecX^{H}* and wildtype *FecX*⁺) from PCR fragments (Galloway *et al.,* 2000), and sequencing was carried out on an ABI 373 sequencer. We confirmed the single base substitutions by sequencing genomic DNA covering the entire coding region from at least six animals carrying each allele ( *FecX^{I}*, *FecX^{H}* and *FecX⁺*). Aside from the *FecX^{H}* or *FecX^{I}* base substitutions (Figures 2a and 2b) only one other variation was detected in the GDF-9B gene in sheep, namely a single Leu codon (CTT) deletion at L10 or L11 in the signal sequence in some animals (refer Figures 4 and 5). The Leu deletion is not associated with either the *FecX^{H}* or *FecX^{I}* alleles, and appears breed-related. The *FecX^{H}* C→T substitution results in loss of a *Bsr*SI restriction site (actg/gn) and gain of a *Spe*I site (a/ctagt). We confirmed this base substitution by demonstrating *Spe*I cleavage of a 541 bp PCR product spanning this region into 476 and 65 bp fragments in *FecX^{H}*/*FecX^{H}* females and *FecX*^{*H*/}*^{Y}* males, but not in *FecX^{I}* and wildtype animals. In sheep carrying a copy of each allele (*FecX^{I}l FecX^{H}*) all three fragments were identified (541, 476 and 65bp). Similarly, *Bsr*SI cleaved fragments occurred for *FecX^{I}* and wildtype animals but not *FecX^{H}* carriers. A 154 bp PCR product from DNA of FecX^{I} carriers (produced from primers:
#12 (GAAGTAACCAGTGTTCCCTCCACCCTTTTCT); and
#13 (CATGATTGGGAGAATTGAGACC));
generated a forced *Xba*I restriction site. *Xba*I (t/ctaga) cleaved PCR products generated from *FecX^{I}*/*FecX^{I}* females and *FecX*^{*I*/}*^{Y}* males carrying the A allele (tctaga), but not wildtype or *FecX^{H}* PCR products carrying the T allele (tctagt). Thus *Xba*I cleaved the 154 bp PCR product to a 124 bp fragment by removing the 30 nucleotide primer #12 only in *FecX^{I}* carriers. All restriction digests were carried out on aliquots of PCR products as specified by the manufacturers and fragments were separated in 3% FMC Metaphor agarose gels.

### Linkage Mapping Methods

We constructed a sheep X-chromosome genetic linkage map by multipoint analysis using CRIMAP as previously described (Galloway *et al.,* 1996) and mapped additional markers *MAOA, McM551, OarMP1*, and *TIMP1* (Galloway *et al.,* 2000). *FecX^{I}* and *GDF-9B* were mapped in the Inverdale linkage mapping families generated by mating nine carrier males (*FecX*^{*I*/*Y*}) to wildtype females to produce 62 heterozygous *FecX^{I}*/*FecX*⁺ female progeny in the second generation. These 62 females produced 96 homozygous *FecX^{I}lFecX^{I}* or heterozygous *FecX^{I}*/*FecX*⁺ female progeny when mated to 10 *FecX*^{*I*/*Y*} males. We determined carrier status of Inverdale animals by laparoscopy to identify *FecX^{I}*/*FecX^{I}* infertile females and by progeny testing and laparoscopy of female offspring to identify *FecX*^{*I*/}*^{Y}* males. Parentage was confirmed with genetic markers and all *FecX^{I}FecX*⁺ females selected in the third generation were full siblings of *FecX^{I}lFecX^{I}* infertile females. No DNA was collected from the wildtype females in the first generation. We mapped *GDF-9B* on the basis of the T→A mutation in the gene coding region.

### RESULTS

### Sequencing results

PCR fragments encoding the entire mature peptide were sequenced from Inverdale and Hanna genomic DNA. The sequenced region also included most of the propeptide in exon 2 (from 70 bases 3' to the human/mouse intron/exon boundary to 30 bases beyond the tga stop codon ). Sequence from these two sheep lines was compared with the wildtype sheep sequence for GDF-9B. Sequence data revealed two distinct single base substitutions within the mature GDF-9B peptide, one segregating within the Inverdale pedigree and one within the Hanna pedigree (Figure 2).

In Hanna animals the C nucleotide at position 67 nucleotides beyond the mature peptide processing site is a T. This converts the codon CAG (coding for the amino acid glutamine (G)) to the codon TAG (coding for termination), and would result in a truncated mature protein (Figure 3b).

In Inverdale animals the T nucleotide at position 92 nucleotides beyond the mature peptide processing site has become an A, converting the codon GTC (amino acid valine (V)) to GAC (amino acid aspartic acid (D)) (Figure 3a).

### Verification of the single base substitutions

These single base substitutions have been verified by sequencing at least 6 animals carrying each genotype (Inverdale, Hanna and non-carrier wildtype). Each animal was sequenced at least once (Table 1). In this subset of animals neither of the Inverdale or Hanna substitutions were seen in wildtype animals, nor was the Inverdale substitution seen in Hanna animals or vice versa.

**Table 1.**

| Sequencing identification of single base substitutions in Inverdale and Hanna animals. | | | | | |
|---|---|---|---|---|---|
| Animals are of known genotype from well-characterised pedigrees (+ = wildtype allele, I = Inverdale allele, H = Hanna allele, Y = Y-chromosome). Numerals indicate the number of times an independent sequence from that animal identified the appropriate sequence variation. | | | | | |
| Genotype | Animal | Hanna | | | Inverdale |
| | ID | Cag (wt) | Tag | gTc (wt) | gAc |
| Y ram | 667 | 2 | | | 2 |
| IY ram | 3432 | 1 | | | 1 |
| II ewe | 2663 | 1 | | | 1 |
| HY ram | 9513 | | 3 | 3 | |
| HY ram | 4864 | | 1 | 1 | |
| HH ewe | 7133 | | 2 | 2 | |
| HI ewe | 7141 | 2 | 2 | 2 | 2 |
| HI ewe | 4865 | 1 | 1 | 1 | 1 |
| H+ ewe | 7151 | 1 | 1 | 1 | |
| I+ ewe | 2682 | 1 | | 1 | 1 |
| +Y Romney | 7610 | 2 | | | 2 |
| ++ Romney | 2884 | 2 | | | 2 |
| ++ Romney | 2958 | 2 | | | 2 |
| +Y Romney | 1079 | 1 | | | 1 |
| +Y Merino | 100 | 2 | | | 2 |
| ++ Merino | 121 | 1 | | | 1 |

A restriction enzyme search revealed that the Hanna base substitution produced a SpeI enzyme cleavage site (a/ctagt) and removed a BsrSI (actg/gn) site around that substitution. These cleavage sites were confirmed by demonstrating that the enzyme Spel was able to cleave a 541bp PCR fragment spanning this region into 476bp and 65bp fragments in HY and HH animals, but not in IY and +Y animals. In a sheep carrying one copy of both the Inverdale and the Hanna genes (HI), both the 541bp and 476bp fragments were identified.

Similarly BsrS1 was shown to cleave fragments from IY and +Y animals but not HY, and the HI sheep showed a mixture of both bands.

No enzyme cleavage sites are generated or removed from around the Inverdale base substitution site, so a forced RFLP primer was generated which introduces an Xba1 cleavage site (t/ctaga) into the PCR product generated from an Inverdale allele, but not a wildtype. The PCR product containing the introduced Xba1 site is only produced when the Inverdale A mutation is present, and is not present in Hanna or wildtype animals. In this case the PCR product is cleaved by Xba1, removing 30 bases, and resulting in a size change in the length of the final product.
Inverdale DNA strand..TTTCAAGACAGCTT..
30b PCR primer ending-tttct
Produces Xba1 cut site tctaga in final PCR product

Using this method PCR fragments from 2 HY, 1 HH, 2 +Y and 3 ++ animals were not cleaved by Xba1, while fragments from 36 II and 12 IY animals were cleaved. One HI and 47 I+ animals showed a mixture of cleaved and uncleaved fragments, as expected for heterozygotes.

### Sequencing and Mutation Detection

We sequenced sheep GDF-9B gene sequences from cDNA and genomic DNA, using primers designed from human, mouse and sheep sequences (Galloway *et al.,* 2000). The sheep gene is similar to human, mouse and rat (Laitinen *et al.,* 1998; Dube *et al*., 1998; Aaltonen *et al.,* 1999; Jaatinen *et al.,* 1999), with gene features typical of other members of the TGFβ superfamily. The full-length 1179 bp sequence encodes a 393 amino acid prepropeptide (Figure 4) spanning two exons separated by an intron of approximately 5.4 kb. A 25 amino acid predicted signal peptide precedes a 244 amino acid proregion and a putative 125 amino acid C-terminal mature peptide region beyond the RRAR protease cleavage site. The sheep coding region is 82.9% homologous with human, 78.8% with mouse and 78.4% with rat at the nucleotide level.

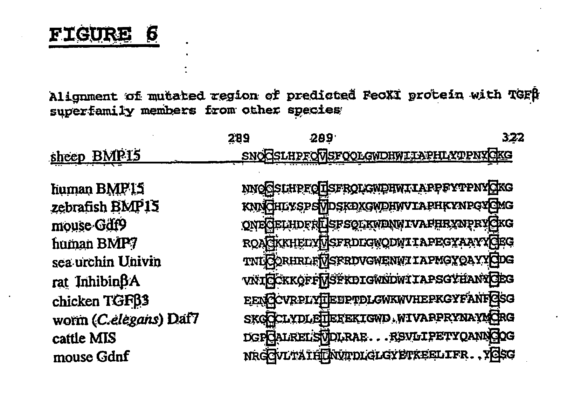

We also sequenced genomic DNA in Inverdale (*FecX^{I}*) and Hanna (*FecX^{H}*) carriers (Figure 5). A single C→T transition at nucleotide position 67 of the mature peptide coding region of *FecX^{H}* carriers introduces a premature stop codon in the place of glutamic acid (Q) at amino acid residue 23 (residue 291 of the unprocessed protein). Premature truncation so early in the mature peptide in *FecX^{H}* carriers is likely to result in complete loss of GDF-9B function. A distinct single T→A transition occurs in *FecX^{I}* carriers at nucleotide position 92 of the mature peptide. The mutation substitutes the valine (V) with aspartic acid (D) at residue 31 (residue 299 of unprocessed protein). The *FecX^{I}* mutation is a non-conservative change in a highly conserved region of the protein. All other members of the TGFβ superfamily from a wide range of species contain only the conserved hydrophobic amino acids valine, isoleucine or leucine at this position (Figure 6).

### Mapping of GDF-9B in sheep

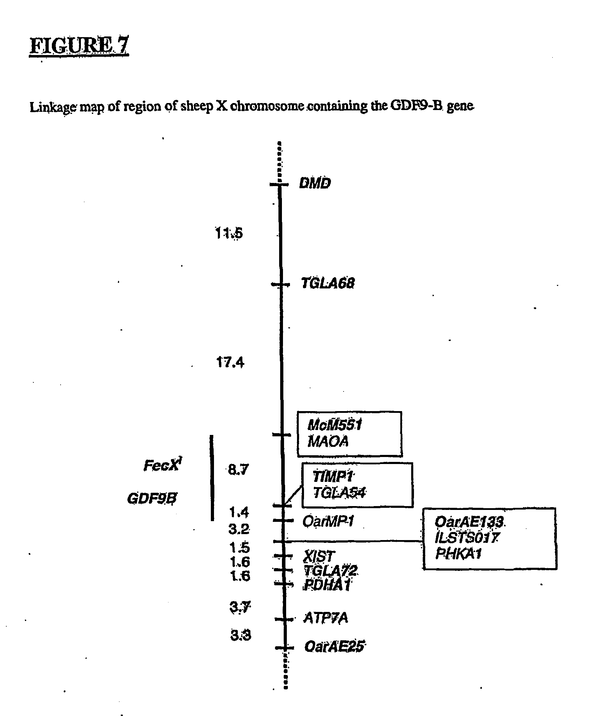

In order to locate *FecX^{I}* we generated a genetic linkage map of the sheep X-chromosome (Galloway *et al.,* 1996), and we have mapped the *FecX^{I}* locus between flanking markers 10 cM apart at the centre of the sheep X-chromosome (Figure 7). Linkage relationships with the Inverdale phenotype were observed in a family of 177 animals in a three-generation structure with a maximum of 96 informative female meioses. Linkage mapping indicated that *FecX^{I}* mapped to a region containing *TIMP1* and *MAOA* (syntenic with human Xp11.2-11.4) and not the region containing *PHKA1, XIST* and *ATP7A* (human Xq13). A breakpoint near *OarMP1* in sheep appears to separate these two groups of genes belonging to distinct syntenic groups on the human and mouse X-chromosomes. *GDF-9B* maps to human Xp11.2 and to a syntenic region of the mouse X-chromosome (Dube *et al*., 1998; Aaltonen *et al*., 1999). We have mapped sheep *GDF-9B* into the same 10 cM interval as *FecX^{I}* in our Inverdale mapping pedigree, and found no recombinants between the *FecX^{I}* phenotype and *BMP15* out of 78 co-informative female meioses refer Table 2.

**Table 2.**

| Linkage of *FecX^{I}* to genes and markers on the sheep X-chromosome | | | | |
|---|---|---|---|---|
| Marker | Number of recombinants | Co-informative meioses | Recombination fraction (θ) | Lod score (female) |
| TGLA68 | 7 | 176 | 0.17 | 4.20 |
| MAOA | 2 | 143 | 0.15 | 1.49 |
| McM551 | 9 | 196 | 0.07 | 11.61 |
| GDF-9B | 0 | 213 | 0 | 23.18 |
| TIMP1 | 0 | 177 | 0 | 12.34 |
| TGLA54 | 0 | 170 | 0 | 10.54 |
| OarMP1 | 1 | 206 | 0.01 | 18.79 |
| ATP7A | 1 | 147 | 0.08 | 2.08 |
| XIST | 2 | 148 | 0.13 | 2.20 |
| PHKA1 | 4 | 176 | 0.08 | 8.47 |
| OarAE133 | 5 | 211 | 0.07 | 14.57 |

CRIMAP Two point linkage analysis to *FecX^{I}* phenotype in Inverdale mapping pedigree

No significant sequence differences were found between the *TIMP1* coding DNA of wildtype and *FecX^{I}* sheep and identification of a subsequent recombinant among additional *FecX^{I}* carriers eliminated *TIMP1* as a candidate for *FecX^{I}.*

### Use of Isolated polypeptide and antibody to manipulate ovulation.

An *E*. *coli* -derived mature protein of GDF-9B comprising a wildtype sequence as set out in SEQ ID No: 10 was chemically conjugated to the protein Keyhole Limpet Haemocyanin (KLH), and this antigen in Freund's Complete Adjuvant (FCA) was injected subcutaneously (sc) into 10 anoestrous Romney ewes (0.4 mg/ewe). A further 9 anoestrous Romney ewes were injected with KLH alone (sc) in FCA to serve as controls (0.4 mg/ewe). Thereafter all animals were injected at monthly intervals with booster antigen (. 0.2 mg/ewe KLH-GDF9B or 0.2 mg/ewe KLH) in a Span, Tween, oil adjuvant. As the ewes entered the breeding season, some 3-4 months after initiating the immunisations, the animals showing oestrous behaviour, as detected by a vasectomised ram with marking harness, were subjected to a laparoscopy procedure to visualise the number of corpora lutea (i.e. ovulation sites) on the surface of the ovaries. Seven of the 10 KLH-GDF-9B treated animals and all of the 9 KLH treated animals showed oestrous behaviour. The mean ovulation rates in the KLH-GDF9B and KLH immunised sheep which showed oestrous activity are shown in Table 3.

**Table 3.**

| | | |
|---|---|---|
| Mean ovulation rate in sheep^{*} showing oestrous activity following repeated immunisation with Keyhole Limpet Haemocyanin (KLH) or KLH conjugated to an *E*. *coli* expressed GDF9B antigen | | |

| Treatment | Geometric mean ovulation rate | Number of sheep showing oestrous activity |
|---|---|---|
| | (95% confidence rate) | |
| KLH | 1.4 (1.2, 1.7) | 9 |
| KLH-GDF9B | 4.5 (2.7,7.5) | 7 |

The KLH-GDF-9B animals showed a highly significant increase in ovulation rate compared to the KLH control animals (p<0.001) ANOVA.

+Evidence that the increased ovulation rate in the KLH-GDF-9B animals that showed oestrus was associated with an antibody response to GDF-9B is shown in Table 4.

**Table 4.**

| | | |
|---|---|---|
| Mean (range) antibody levels in sheep plasma before or after repeated immunisation of female sheep with KLH or KLH conjugated to an E. coli-derived GDF-9B mature peptide. The values presented show the absorbance at 490 nm which represents the levels of antibody to GDF-9B | | |

| Treatment | Preimmune | Immune |
|---|---|---|
| KLH | <0.3 | <0.3 |
| KLH-GDF-9B (*E*. *coli* | <0.3 | 1.932 |
| expressed mature protein) | | (1.454-2.613) |

Antibody levels were measured by an ELISA procedure after the sheep plasmas were diluted 1:5000. The ELISA method involved coating a 96-well plate with 100 ng/well of an *E*. *coli* expressed full-length GDF-9B and incubation with 100 µl of diluted sheep plasma and 100 µl of assay buffer, after appropriate blocking treatment and successive washes. After incubation with the sheep plasma and several washes, rabbit anti-sheep-HRP was added for 1 h at 37°C. The wells were then washed and developed with o-phenylenediamine plus hydrogen peroxide with development being stopped with sulphuric acid.

In a separate study to demonstrate that a functional variant of ovine GDF-9B will influence ovarian follicular development, 10 female mice were immunised intraperitoneally (ip) with an *E*. *coli* -derived mature ovine GDF-9B protein (0.2 mg) in FCA (0.22 ml), and another 10 female mice were immunised with bovine alpha lactalbumin (0.2 mg) in FCA (0.22 ml ip) to serve as controls. Subsequently, 3 booster injections of the appropriate antigens (0.1 mg at first booster and 0.05 mg at second and third booster) were given at 2 week intervals in a Span/Tween/oil mixture and the animals sacrificed 1 week after the final booster. Thereafter the ovaries were fixed in Bouin's aqueous fixative and processed for morphometric analysis. The total number of growing ovarian preantral and antral follicles was determined using a systematic random sampling procedure. The data are summarised in Table 5.

**Table 5.**

| | | |
|---|---|---|
| Mean numbers of preantral and antral follicles in mouse ovaries following immunisation with ovine GDF9B or bovine alpha lactalbumin | | |

| Treatment | Preantral or antral follicles | Geometric mean number of follicles |
|---|---|---|
| | | (95% confidence limits) |
| Bovine α-lactalbumin | Preantral | 329 (291,371) |
| | Antral | 80 (55, 115) |
| GDF9B | Preantral | 261 (233, 292) |
| | Antral | 84 (57,124) |

The number of preantral follicles in the GDF9B treated animals was significantly lower than that in the bovine α-lactalbumin treated mice, p<0.005 (ANOVA). There were no significant differences between the treatment groups with respect to the number of antral follicles.

Evidence that the differences in number of preantral follicles was associated with an antibody response to GDF-9B is as follows. The mean (range) antibody level in mouse serum diluted 1:50,000, following repeated immunisation was 2.18 (1.28-2.90) whereas all mice immunised with α-lactalbumin had no response (i.e. <0.1). The antibody values, represented by the absorbance at 490 nm, were measured by an ELISA procedure.

In a further study we induced sterility in recipient animals by the administration of an antigen corresponding to an ovine GDF-9B peptide sequence. To achieve sterility a 15-mer amino acid peptide sequence corresponding to a variant of the mutated and wildtype ovine GDF-9B mature region was synthesised together with a C-terminal cysteine for conjugation to Keyhole Limpet Haemocyanin (KLH) to generate the antigen. The peptide sequence we utilised was: SEVPGPSREHDGPESC. In this study, 10 anoestrous Romney ewes were injected with 0.4 mg/ewe of the KLH-GDF-9B peptide antigen in Freund's complete adjuvant, and 9 anoestrous Romney ewes were injected with 0.4 mg/ewe KLH antigen as a control group. Subsequently at monthly intervals on 6 occasions, the animals were boosted with further antigen (0.2 mg/ewe on each occasion) in a Span/Tween/oil mixture (sc) and oestrous activity monitored 2-3 times weekly using vasectomised rams. The ovulation rate as assessed by laparoscopy was examined around 1 week before the final booster treatment.

All 9 KLH treated ewes displayed regular cyclical oestrous activity, whereas only 1 out of the 10 KLH-GDF-9B peptide treated animals showed oestrous activity. The geometric mean (and 95% confidence limits) for ovulation rate in the KLH control animals was 1.5 (1.1, 1.9), whereas in the 9 KLH-GDF-9B peptide treated animals which did not show oestrous the ovulation rate was zero. In the one KLH-GDF-9B peptide treated animal displaying oestrous activity, the ovulation rate was 5. These data unequivocally show that sterility can be induced by the administration of antibody or mutated GDF-9B antigen or a variant thereof.

Evidence to support the claim that the induction of an ovulation in the 9 KLH-GDF-9B (16 mer) (i.e. 15 mer + c-terminal cysteine) peptide refer SEQ ID NO: 11 treated animals which did not show oestrus was associated with an antibody response to GDF-9B is shown in Table 3c.

**Table 6.**

| | | |
|---|---|---|
| Mean (range) antibody levels in sheep plasma before or after repeated immunisation of female sheep with KLH or KLH conjugated to a GDF-9B 16 mer peptide. The values presented show the absorbance at 490 nm which represents the level of antibody to GDF-9B | | |

| Treatment | Preimmune | Immune |
|---|---|---|
| KLH | <0.3 | <0.3 |
| KLH-GDF-9B (16 mer peptide) | <0.3 | 2.392 |
| | | (1.085-3.000) |

Antibody levels were measured by an ELISA procedure as summarised for Table 4.

Collectively these results demonstrate that by administering a GDF-9B antigen the resultant production of antibody in the recipient animals may lead to altered ovarian follicular activity and thus effect modulation of the ovulation rate.

### DNA test for mutations.

Sequence variants in the gene for GDF-9B can be determined by a variety of methods, well known to researchers skilled in the art, which are specifically designed to identify differences between alleles of the gene. In particular these methods can be used to identify the Inverdale (*FecX^{I}*) and Hanna (*FecX^{H}*) single nucleotide polymorphisms (SNPs), namely the C→T transition in *FecX^{H}* carriers and the T→A transition occurs in *FecX^{I}* carriers, but such methods can also be applied to other alleles of this gene which may be present in other mammals. Samples can be obtained either from DNA or directly from punches of whole blood spotted directly onto FTA® paper or from hair or wool follicles.

One such method involves the use of restriction enzymes to cleave the DNA specifically for one allele and not the other, or to cleave t a PCR fragment containing a primer which has been designed to contain a cleavage site in combination with one allele or the other.

The *FecX^{H}* C→T substitution results in loss of a *Bsr*SI restriction site (actg/gn) and gain of a *SpeI* site (a/ctagt). We confirmed this base substitution by demonstrating *SpeI* cleavage of a 541 bp PCR product spanning this region into 476 and 65 bp fragments in *FecX^{H}lFecX^{H}* females and *FecX*^{*H*/}*^{Y}* males, but not in *FecX^{I}* and wildtype animals. In sheep carrying a copy of each allele (*FecX^{I}*/ *FecX^{H}*) all three fragments were identified (541, 476 and 65bp). Similarly, *BsrSI* cleaved fragments from *FecX^{I}* and wildtype animals but not *FecX^{H}* carriers.

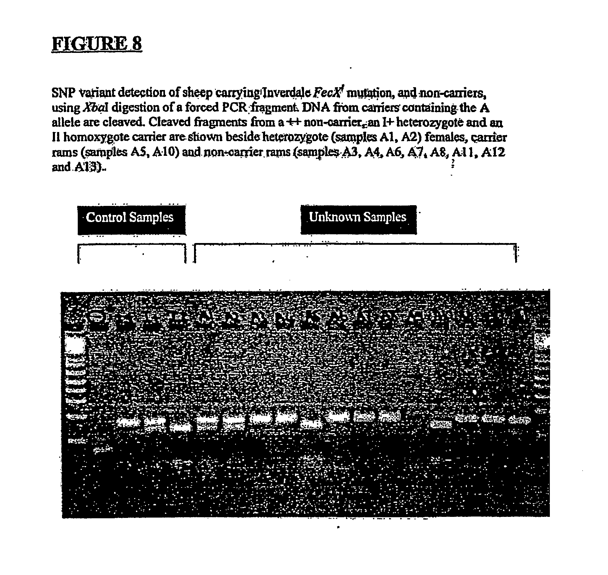

A 154 bp PCR product from DNA of *FecX^{I}* carriers (produced from primers:
#12 (GAAGTAACCAGTGTTCCCTCCACCCTTTTCT); and
#13 (CATGATTGGGAGAATTGAGACC))
generated a forced *Xba*I restriction site. *Xba*I (t/ctaga) cleaved PCR products generated from *FecX^{I}*/*FecX^{I}* females and *FecX*^{*I*/}*^{Y}* males carrying the A allele (tctaga), but not wildtype or *FecX^{H}* PCR products carrying the T allele (tctagt). Thus *Xba*I cleaved the 154 bp PCR product to a 124 bp fragment by removing the 30 nucleotide primer #12 only in *FecX^{I}* carriers Figure 8.

Products were detected by electrophoresis in 3% FMC Metaphor agarose gels containing ethidium and visualised under ultraviolet light.

Another SNP detection method includes the use of fluoroescently-labelled primers in conjunction with the forced RFLP method above, and visualising the products on a sequencing machine such as the ABI377.

Other methods for SNP detection include the use of either the Taqman® Allelic Discrimination method or the SnaPshot™ ddNTP Primer Extension Kit (insert manufacturers details here). The Taqman allelic discrimination employs a probe technology that exploits the 5'-3' nuclease activity of AmpliTaq Gold® DNA polymerase to allow direct detection of the PCR product by the release of a fluorescent reporter as a result of PCR. Two probes are used in the allelic discrimination assay, one probe for each allele, with each probe containing a different reporter dye. The SnaPshot system is based on the dideoxy single nucleotide (fluoroescently labelled) extension of an unlabelled oligonucleotide primer for the detection of single nucleotide polymorphisms (SNPs). Another SNP detection method employs mass spectrometry whereby the region around the SNP or mutation is amplified by PCR and an oligonucleotide primer is extended through the SNP or mutation in the presence of dideoxynucleotides. SNP variants are detected on the basis of mass difference.

### REFERENCES

Aaltonen, J., Laitinen, M. P., Vuojolainen, K., Jaatinen, R., Horelli-Kuitunen, N., Seppa, L., Louhio, H., Tuuri, T., Sjoberg, J., Butzow, R., Hovata, O., Dale, L. and Ritvos, O. (1999) Human growth differentiation factor 9 (GDF-9) and its novel homolog GDF-9b are expressed in oocytes during early folliculogenesis. J. Clin Endocrinol Metab 84: 2744-2750
Amer, P.R., McEwan, J.C., Dodds, K.G. and Davis, G.H. 1998: Cost benefit analysis of commercial use of the Inverdale prolificacy gene in sheep. Proceedings of the New Zealand Society of Animal Production 58: 157-160.
Braw-Tal, R., McNatty, K.P., Smith, P., Heath, D.A., Hudson, N.L., Phillips, D.J., McLeod, B.J. and David, G.H. 1993: The ovaries of ewes homozygous for the X-linked Inverdale gene (FecX1) are devoid of secondary and tertiary follicles but contain many abnormal structures. Biology of Reproduction 49: 895-907.
Davis, G.H., McEwan, J.C., Fennessy, P.F., Dodds, K.G. and Farquhar, P.A. 1991: Evidence for the presence of a major gene influencing ovulation rate on the X-chromosome of sheep. Biology of Reproduction 44: 620-624.
Davis, G.H., McEwan, J.C., Fennessy, P.F., Dodds, K.G., McNatty, K.P. and O, W-S. 1992: Infertility due to bilateral ovarian hypoplasia in sheep homozygous (FecXI FecXI) for the Inverdale prolificacy gene located on the X-chromosome. Biology of Reproduction 46: 636-640.
Davis, G.H., Bruce, G.D. and Reid, P.J. 1994: Breeding implications of the streak ovary condition in homozygous (FecXI FecXI) Inverdale sheep. Proceedings of the 5th World Congress on Genetics Applied to Livestock Production. 19: 249-252.
Davis, G.H., McEwan, J.C., Fennessy, P.F., Dodds, K.G. 1995: Discovery of the Inverdale gene (FecX). Proceedings of the New Zealand Society of Animal Production 55: 289-290.
Dong J., Albertini D.F., Nishimori K., Rajendra Kumar T., Lu N. & Matzuk M.M. 1996: Growth differentiation factor-9 is required during early ovarian folliculogenesis. Nature 383: 531-535.
Dube, J.L., Wang, P., Elvin, J., Lyons, K.M., Celeste, A.J. and matzuk, M.M. 1998: The bone morphogenic protein 15 gene is X-linked and expressed in oocytes. Molecular Endocrinology 12: 1809-1817.
Galloway, S.M., Hanrahan, V., Dodds, K.G., Potts, M.D., Crawford, A.M. and Hill, D.F. 1996: A linkage map of the ovine X chromosome. Genome Research 6: 667-677.
Galloway, S.M., Cambridge, L.M., Henry, H.H., van Stijn, T.C. and Davis, G.H. 1999: A genetic test to identify carriers of the ovine Inverdale fecundity gene. Proceedings of the New Zealand Society of Animal Production 59: 114-116.
Galloway, S.M., McNatty, K.P., Cambridge, L.M., Laitinen, M.P.E., Juengel. J.L., Jokiranta, T.S., McLaren, R.J., Luiro, K., Dodds, K.G., Montgomery, G.W., Beattie, A.E., Davis, G.H., and Ritvos, O. (2000) Mutations in an oocyte-derived growth factor gene (BMP15) cause increased ovulation rate and infertility in a dosage-sensitive manner. Nature Genetics 25: 279-283
Gray, A.J. and Davis, G.H. Commercial performance of sheep carrying the Inverdale gene (FecX). Proceedings of the New Zealand Society of Animal Production 55: 294-295.
Hanna, M.M. 1995: Living with the Inverdale gene (FecX) in a Romney flock. Proceedings of the New Zealand Society of Animal Production 55: 296-297.
Jaatinen, R., Laitinen, M.P., Vuojolainen, K., Aaltonen, J., Louhio, H., Heikinheimo, K., Lehtonen, E. and Ritvos, O. (1999) Localisation of growth differentiation factor-9 (Gdf-9) mRNA and protein in rat ovaries and cDNA cloning of rat GDF-9 and its novel homolog GDF-9B.Mol Cell Endocrinol 156: 189-193
Laitinen, M, Vuojolainen, K., Jaatinen, R., Ketola, I., Aaltonen, J., Lehtonen, E., Heikinheimo, M. and Ritvos, O. 1998: A novel growth differentiation factor-9 (GDF-9) related factor is co-expressed with GDF-9 in mouse oocytes during folliculogenesis. Mechanisms of Development 78: 135-140.
McNatty, K.P., Smith, P., Hudson, N.L., Heath, D.A., Tisdall, D.J., O, W-S., Braw-Tal, R. 1995: Development of the sheep ovary during foetal and early neonatal life and the effect of fecundity genes. Journal of Reproduction and Fertility, Supplement 49: 123-135.
McNatty, K.P., Smith, P., Hudson, N.L., Lun, S., Heath, D., Shackell, G and Corrigan, K. 1995: Ovarian characteristics in Inverdale ewes heterozygous (I+) and homozygous (II) for the Inverdale gene (FecX). Proceedings of the New Zealand Society of Animal Production 55: 301-303.
Montgomery, G.W. and Sise, J.A. 1990: Extraction of DNA from sheep white blood cells. New Zealand Journal of Agricultural Research 33: 437-441.
Mouse Genome Database (MGD), Mouse Genome Informatics, The Jackson Laboratory, Bar Harbor, Maine (October 1999). World Wide Web (URL: http://www.informatics.jax.org/).
Ohno, S. 1973: Ancient linkage groups and frozen accidents. Nature 244: 259-262.
Sadighi, M., Montgomery, G.W., Bodensteiner, K.J., and Galloway, S.M. 1998: The growth differentiation factor-9 maps to sheep chromosome 5. Proceedings of the 26th International Conference on Animal Genetics, Auckland, New Zealand, abstract C020.
Smith, P., O, W-S., Corrigan, K.A., Smith, T., Lundy, T., David, G.H. and McNatty, K.P. 1997: Ovarian morphology and endocrine characteristics of female sheep foetuses that are heterozygous or homozygous for the Inverdale prolificacy gene (FecX1). Biology of Reproduction 57: 1183-1192.

## Claims

1. An isolated mutated GDF-9B nucleic acid molecule encoding a mutated polypeptide capable of modulating the ovulation rate of a female mammal, said nucleic acid molecule comprising a nucleotide sequence selected from the group consisting of:
a) SEQ ID NO: 1, SEQ ID NO: 3, SEQ ID NO: 5, or SEQ ID NO: 7; and
b) a sequence complementary to the molecule(s) defined in (a).

2. A vector comprising a nucleic acid molecule(s) of claim 1.

3. A construct comprising the nucleic acid molecule(s) of claim 1.

4. A host cell which has been transformed by a vector or construct as claimed in claim 2 or 3.

5. An isolated mutated polypeptide capable of modulating the ovulation rate of a female mammal, wherein said polypeptide is encoded by a nucleic acid molecule as claimed in claim 1.

6. An isolated mutated polypeptide capable of modulating the ovulation rate of a female mammal, comprising an amino acid sequence of SEQ ID NO: 2 or SEQ ID NO: 6.

7. An isolated mutated polypeptide capable of modulating the ovulation rate of a female mammal consisting of the sequence of SEQ ID NO: 4.

8. An isolated nucleic acid molecule encoding a polypeptide as claimed in claim 6 or 7.

9. A method of identifying a mammal which carries a mutated nucleic acid molecule encoding GDF-9B, said method comprising the steps of:
(i) isolating DNA from a mammal tissue or blood sample; and optionally
(ii) isolating GDF-9B DNA from DNA obtained at step (i);
(iii) probing said DNA with a probe complementary to the mutated GDF-9B DNA of claim 1(a) or 8;
(iv) amplifying the amount of mutated GDF-9B DNA; and/or
(v) determining whether the GDF-9B sequence DNA obtained in Step (iv) carries a mutation associated with sterility, or increased or decreased ovulation.

10. A method according to claim 9, in which the mammal is male or female, and carries a single copy of the mutated GDF-9B nucleic acid molecule.

11. A method according to claim 10, in which the mammal is female, and carries two copies of the mutated GDF-9B nucleic acid molecule.

12. A polypeptide as claimed in claim 6 or 7 for use as a medicament.

13. A polypeptide as claimed in claim 6 or 7 for use in increasing the ovulation rate of a female mammal which does not carry a mutated GDF-9B nucleic acid molecule.

14. An agent comprising an immunising effective amount of a mutated GDF-9B polypeptide comprising an amino acid sequence as claimed in claim 6 or 7 for use in increasing or reducing the ovulation rate or inducing sterility in a female mammal by inhibiting the biological activity of the mutated GDF-9B polypeptide.

15. A composition comprising an effective amount of a polypeptide as claimed in claim 6 or 7 together with a pharmaceutically or veterinarily acceptable carrier or diluent.

16. A composition containing an effective amount of an agent comprising a mutated GDF-9B polypeptide comprising an amino acid sequence as claimed in claim 6 or 7 together with a pharmaceutically or veterinarily acceptable carrier or diluent.

17. The use of a nucleic acid molecule as claimed in claim 1 to identify sequence variants in an individual mammal associated with increased ovulation, reduced ovulation or the sterility of that mammal.

18. A kit for identifying mammals which carry the mutated GDF-9B nucleic acid molecules of claim 1, said kit comprising;
a) primer pairs for amplification of the appropriate region of the GDF-9B; and optionally one or more of the following
b) buffer solution for the DNA amplification;
c) a mixture of deoxynucleotides;
d) means for DNA amplification;
e) control DNA from the species being tested;
f) appropriate standards; and
g) a detection system.

19. An isolated nucleic acid molecule encoding a mutated polypeptide capable of modulating the ovulation rate in a female mammal, said nucleic acid molecule comprising a nucleic acid sequence as set forth in SEQ ID NO: 12 or SEQ ID NO: 14.

20. An isolated mutated polypeptide capable of modulating the ovulation rate of a female mammal, comprising the sequence as set forth in SEQ ID NO: 13

21. A nucleic acid as claimed in claim 19 or a polypeptide as claimed in claim 20 for use as a medicament.

22. A nucleic acid as claimed in claim 19 or a polypeptide as claimed in claim 20 for use in modulating the ovulation rate of a female mammal.

23. A use of a polypeptide as claimed in claim 6 or 7 in the manufacture of a medicament for modulating the ovulation rate of a female mammal, in need thereof.

24. A use of a polypeptide as claimed in claim 6 or 7 in the manufacture of a medicament for increasing the ovulation rate of a female mammal in need thereof, wherein said female mammal does not carry a mutated GDF-9B nucleic acid molecule.

25. A use, in the manufacture of a medicament for increasing or reducing the ovulation rate or inducing sterility in a female mammal, of a mutated GDF-9B polypeptide comprising an amino acid sequence as claimed in claim 6 or 7.

26. A use of an isolated GDF-9B nucleic acid molecule as claimed in claim 1 in the manufacture of a medicament for modulating the ovulation rate of a female mammal.

27. A use as claimed in any one of claims 23 to 26, wherein the female mammal is selected from the group consisting of: humans, sheep, cattle, goats, deer, horses, camelids, possums, pigs, mice, rats, rabbits, hares, weasels, ferrets, cats and dogs.

## Patentansprüche

1. Isoliertes mutiertes GDF-9B-Nukleinsäuremolekül, das ein mutiertes Polypeptid kodiert, das in der Lage ist, die Ovulationsrate eines weiblichen Säugetiers zu modulieren, wobei das Nukleinsäuremolekül eine Nukleotidsequenz umfasst, die ausgewählt ist aus der Gruppe bestehend aus:
a) SEQ-ID-NR: 1, SEQ-ID-NR. 3, SEQ-ID-NR. 5 oder SEQ-ID-NR: 7; und
b) einer zu dem/den in a) definierten Molekül(en) komplementäre Sequenz.

2. Vektor umfassend (ein) Nukleinsäuremolekül(e) nach Anspruch 1.

3. Konstrukt umfassend das/die Nukleinsäuremolekül(e) nach Anspruch 1.

4. Wirtszelle, die durch einen Vektor oder ein Konstrukt nach Anspruch 2 oder 3 transformiert wurde.

5. Isoliertes mutiertes Polypeptid, das in der Lage ist, die Ovulationsrate eines weiblichen Säugetiers zu modulieren, wobei das Polypeptid durch ein Nukleinsäuremolekül nach Anspruch 1 codiert ist.

6. Isoliertes mutiertes Polypeptid, das in der Lage ist, die Ovulationsrate eines weiblichen Säugetiers zu modulieren, umfassend eine Aminosäuresequenz der SEQ-ID-NR: 2 oder SEQ-ID-NR: 6.

7. Isoliertes mutiertes Polypeptid, das in der Lage ist, die Ovulationsrate eines weiblichen Säugetiers zu modulieren, bestehend aus der Sequenz SEQ-ID-NR: 4.

8. Isoliertes Nukleinsäuremolekül, das ein Polypeptid nach Anspruch 6 oder 7 codiert.

9. Verfahren zur Identifikation eines Säugetiers, welches ein mutiertes Nukleinsäuremolekül trägt, das GDF-9B codiert, wobei das Verfahren die Schritte umfasst:
(i) Isolieren von DNA aus Gewebe oder einer Blutprobe eines Säugetiers; und optional
(ii) Isolieren der GDF-9B-DNA aus der in Schritt (i) erhaltenen DNA;
(iii) Sondieren der DNA mit einer zu der mutierten GDF-9B-DNA nach Anspruch 1a) oder 8 komplementären Sonde;
(iv) Amplifizieren der Menge an mutierter GDF-9B-DNA; und/oder
(v) Bestimmen, ob die in Schritt (iv) erhaltene GDF-9B-Sequenz-DNA eine mit Sterilität oder erhöhter oder verringerter Ovulation in Verbindung stehende Mutation trägt.

10. Verfahren nach Anspruch 9, wobei das Säugetier männlich oder weiblich ist und eine einzige Kopie des mutierten GDF-9B-Nukleinsäuremoleküls trägt.

11. Verfahren nach Anspruch 10, wobei das Säugetier weiblich ist und zwei Kopien des mutierten GDF-9B-Nukleinsäuremoleküls trägt.

12. Polypeptid nach Anspruch 6 oder 7 zur Verwendung als ein Medikament.

13. Polypeptid nach Anspruch 6 oder 7 zur Verwendung zur Erhöhung der Ovulationsrate eines weiblichen Säugetiers, welches kein mutiertes GDF-9B-Nukleinsäuremolekül trägt.

14. Mittel umfassend eine zur Immunisierung wirksame Menge eines mutierten GDF-9B-Polypeptids umfassend eine Aminosäuresequenz nach Anspruch 6 oder 7 zur Verwendung zur Erhöhung oder Verringerung der Ovulationsrate oder zur Auslösung von Sterilität in einem weiblichen Säugetier durch Inhibieren der biologischen Aktivität des mutierten GDF-9B-Polypeptids.

15. Zusammensetzung umfassend eine wirksame Menge eines Polypeptids nach Anspruch 6 oder 7 zusammen mit einem pharmazeutisch oder veterinärmedizinisch verträglichen Träger oder Verdünnungsmittel.

16. Zusammensetzung enthaltend eine wirksame Menge eines Mittels, das ein eine Aminosäuresequenz nach Anspruch 6 oder 7 umfassendes mutiertes GDF-9B-Polypeptid umfasst, zusammen mit einem pharmazeutisch oder veterinärmedizinisch verträglichen Träger oder Verdünnungsmittel.

17. Verwendung eines Nukleinsäuremoleküls nach Anspruch 1 zur Identifikation von Sequenzvarianten in einem einzelnen Säugetier, die mit erhöhter Ovulation, reduzierter Ovulation oder der Sterilität des Säugetiers in Verbindung stehen.

18. Kit zur Identifikation von Säugetieren, welche die mutierten GDF-9B-Nukleinsäuremoleküle nach Anspruch 1 tragen, wobei das Kit umfasst:
a) Primerpaare zur Amplifikation des entsprechenden Bereichs für GDF-9B; und optional eines oder mehrere der folgenden
b) Pufferlösung für die DNA-Amplifikation;
c) ein Gemisch von Desoxynukleotiden;
d) Mittel zur DNA-Amplifikation;
e) Kontroll-DNA von der zu testenden Spezies;
f) geeignete Standards;
g) und ein Erfassungssystem.

19. Isoliertes Nukleinsäuremolekül, das ein mutiertes Polypeptid codiert, das in der Lage ist, die Ovulationsrate eines weiblichen Säugetiers zu modulieren, wobei das Nukleinsäuremolekül eine in SEQ-ID-NR: 12 oder SEQ-ID-NR: 14 dargestellte Nukleinsäuresequenz umfasst.

20. Isoliertes mutiertes Polypeptid, das in der Lage ist, die Ovulationsrate eines weiblichen Säugetiers zu modulieren, umfassend die in SEQ-ID-NR: 13 dargestellte Sequenz.

21. Nukleinsäure nach Anspruch 19 oder Polypeptid nach Anspruch 20 zur Verwendung als ein Medikament.

22. Nukleinsäure nach Anspruch 19 oder Polypeptid nach Anspruch 20 zur Verwendung zur Modulierung der Ovulationsrate eines weiblichen Säugetiers.

23. Verwendung eines Polypeptids nach Anspruch 6 oder 7 in der Herstellung eines Medikaments zur Modulierung der Ovulationsrate eines weiblichen Säugetiers mit einem entsprechenden Bedarf.

24. Verwendung eines Polypeptids nach Anspruch 6 oder 7 in der Herstellung eines Medikaments zur Erhöhung der Ovulationsrate eines weiblichen Säugetiers mit einem entsprechenden Bedarf, wobei das weibliche Säugetier kein mutiertes GDF-9B-Nukleinsäuremolekül trägt.

25. Verwendung eines mutierten GDF-9B-Polypeptids umfassend eine Aminosäuresequenz nach Anspruch 6 oder 7 in der Herstellung eines Medikaments zur Erhöhung oder Verringerung der Ovulationsrate oder zur Auslösung der Sterilität in einem weiblichen Säugetier.

26. Verwendung eines isolierten GDF-9B-Nukleinsäuremoleküls nach Anspruch 1 in der Herstellung eines Medikaments zur Modulierung der Ovulationsrate eines weiblichen Säugetiers.

27. Verwendung nach einem Ansprüche 23 bis 26, wobei das weibliche Säugetier ausgewählt ist aus der Gruppe bestehend aus: Menschen, Schafen, Rindern, Ziegen, Hirschen, Pferden, Kamelen, Possums, Schweinen, Mäusen, Ratten, Kaninchen, Hasen, Wieseln, Frettchen, Katzen und Hunden.

## Revendications

1. Molécule d'acide nucléique de GDF-9B muté isolé codant pour un polypeptide muté capable de moduler le taux d'ovulation d'un mammifère femelle, ladite molécule d'acide nucléique comprenant une séquence nucléotidique choisie dans le groupe constitué de :
a) SEQ ID NO : 1, SEQ ID NO : 3, SEQ ID NO : 5 ou SEQ ID NO : 7 ; et
b) une séquence complémentaire de la/des molécule(s) définie(s) dans (a).

2. Vecteur comprenant une/des molécule(s) d'acide nucléique selon la revendication 1.

3. Construction comprenant la/les molécule(s) d'acide nucléique selon la revendication 1.

4. Cellule hôte qui a été transformée par un vecteur ou une construction selon la revendication 2 ou 3.

5. Polypeptide muté isolé capable de moduler le taux d'ovulation d'un mammifère femelle, ledit polypeptide étant codé par une molécule d'acide nucléique selon la revendication 1.

6. Polypeptide muté isolé capable de moduler le taux d'ovulation d'un mammifère femelle, comprenant une séquence d'acides aminés de SEQ ID NO : 2 ou SEQ ID NO : 6.

7. Polypeptide muté isolé capable de moduler le taux d'ovulation d'un mammifère femelle constitué de la séquence de SEQ ID NO : 4.

8. Molécule d'acide nucléique isolé codant pour un polypeptide selon la revendication 6 ou 7.

9. Procédé d'identification d'un mammifère qui porte une molécule d'acide nucléique muté codant pour GDF-9B, ledit procédé comprenant les étapes consistant à :
(i) isoler l'ADN à partir d'un échantillon de tissu ou de sang de mammifère ; et éventuellement
(ii) isoler l'ADN de GDF-9B à partir de l'ADN obtenu à l'étape (i) ;
(iii) sonder ledit ADN avec une sonde complémentaire de l'ADN de GDF-9B muté selon la revendication 1 (a) ou 8 ;
(iv) amplifier la quantité d'ADN de GD-9B muté ; et/ou
(v) déterminer si la séquence de l'ADN de GDF-9B obtenue dans l'étape (iv) porte une mutation associée à la stérilité, ou à une augmentation ou une diminution de l'ovulation.

10. Procédé selon la revendication 9, dans lequel le mammifère est un mâle ou une femelle, et porte une copie unique de la molécule d'acide nucléique de GDF-9B muté.

11. Procédé selon la revendication 10, dans lequel le mammifère est une femelle, et porte deux copies de la molécule d'acide nucléique de GDF-9B muté.

12. Polypeptide selon la revendication 6 ou 7, pour une utilisation en tant que médicament.

13. Polypeptide selon la revendication 6 ou 7, pour une utilisation dans l'augmentation du taux d'ovulation d'un mammifère femelle qui ne porte pas une molécule d'acide nucléique de GDF-9B muté.

14. Agent comprenant une quantité immunisante efficace d'un polypeptide de GDF-9B muté comprenant une séquence d'acides aminés selon la revendication 6 ou 7, pour une utilisation dans l'augmentation ou la réduction du taux d'ovulation ou dans l'induction de la stérilité chez un mammifère femelle par l'inhibition de l'activité biologique du polypeptide de GDF-9B muté.

15. Composition comprenant une quantité efficace d'un polypeptide selon la revendication 6 ou 7, conjointement avec un support ou un diluant acceptable d'un point de vue pharmaceutique ou vétérinaire.

16. Composition contenant une quantité efficace d'un agent comprenant un polypeptide de GDF-9B muté comprenant une séquence d'acides aminés selon la revendication 6 ou 7, conjointement avec un support ou un diluant acceptable d'un point de vue pharmaceutique ou vétérinaire.

17. Utilisation d'une molécule d'acide nucléique selon la revendication 1, pour identifier des variants de séquence chez un mammifère individuel associés à une augmentation de l'ovulation, une réduction de l'ovulation ou à la stérilité de ce mammifère.

18. Kit pour identifier des mammifères qui portent les molécules d'acide nucléique de GDF-9B muté selon la revendication 1, ledit kit comprenant :
a) des paires d'amorces pour l'amplification de la région appropriée de GDF-9B ;
et éventuellement un ou plusieurs des composants suivants
b) une solution tampon pour l'amplification de l'ADN ;
c) un mélange de désoxynucléotides ;
d) un moyen pour l'amplification de l'ADN ;
e) un ADN contrôle issu de l'espèce testée ;
f) des étalons appropriés ; et
g) un système de détection.

19. Molécule d'acide nucléique isolé codant pour un polypeptide muté capable de moduler le taux d'ovulation chez un mammifère femelle, ladite molécule d'acide nucléique comprenant une séquence d'acide nucléique telle que représentée par SEQ ID NO : 12 ou SEQ ID NO : 14.

20. Polypeptide muté isolé capable de moduler le taux d'ovulation d'un mammifère femelle, comprenant la séquence telle que représentée par SEQ ID NO : 13.

21. Acide nucléique selon la revendication 19 ou polypeptide selon la revendication 20, pour une utilisation en tant que médicament.

22. Acide nucléique selon la revendication 19 ou polypeptide selon la revendication 20, pour une utilisation dans la modulation du taux d'ovulation d'un mammifère femelle.

23. Utilisation d'un polypeptide selon la revendication 6 ou 7, dans la fabrication d'un médicament destiné à la modulation du taux d'ovulation d'un mammifère femelle, en ayant besoin.

24. Utilisation d'un polypeptide selon la revendication 6 ou 7, dans la fabrication d'un médicament destiné à l'augmentation du taux d'ovulation d'un mammifère femelle en ayant besoin, où ledit mammifère femelle ne porte pas une molécule d'acide nucléique de GDF-9B muté.

25. Utilisation, dans la fabrication d'un médicament destiné à l'augmentation ou à la réduction du taux d'ovulation ou à l'induction d'une stérilité chez un mammifère femelle, d'un polypeptide de GDF-9B muté comprenant une séquence d'acides aminés selon la revendication 6 ou 7.

26. Utilisation d'une molécule d'acide nucléique de GDF-9B isolé selon la revendication 1, dans la fabrication d'un médicament destiné à la modulation du taux d'ovulation d'un mammifère femelle.

27. Utilisation selon l'une quelconque des revendications 23 à 26, où le mammifère femelle est choisi dans le groupe constitué : des êtres humains, des moutons, des bovins, des chèvres, des cerfs, des chevaux, des camélidés, des opossums, des porcs, des souris, des rats, des lapins, des lièvres, des belettes, des furets, des chats et des chiens.


### SEQUENCE LISTING

<110> Agresearch Limited
   Galloway, Susan
   McNatty, Kenneth
   Davis, George
   Ritvos, Olli
<120> Nucleotide sequences involved in increasing or decreasing mammalian ovulation rate
<130> 30929X144
<150> NZ 500844
   <151> 2000-05-05
<160> 17
<170> PatentIn version 3.0
<210> 1
   <211> 778
   <212> DNA
   <213> Ovis aries
<220>
   <221> CDS
   <222> (1) .. (762)
<220>
   <221> mat_peptide
   <222> (388) .. ()
<220>
   <221> mutation
   <222> (479)..(479)
   <223> Inverdale nucleotide a but wildtype nucleotide t, Inverdale codon gac but wildtype codon gt
<220>
   <221> misc_feature
   <222> (376)..(387)
   <223> furin protease sequence
<220>
   <221> misc_feature
   <222> (763)..(765)
   <223> stop codon
<400> 1
   ctt cac cta act cat  tcc cac ctc tcc tgc  cat gtg gag ccc tgg          45
   Leu His Leu Thr His  Ser His Leu Ser Cys  His Val Glu Pro Trp
                   -125                 -120                 -115
   gtc cag aaa agc cca  acc aat cac ttt cct  tct tca gga aga ggc          90
   Val Gln Lys Ser Pro  Thr Asn His Phe Pro  Ser Ser Gly Arg Gly
                     -110                 -105                -100
   tcc tca aag cct tcc ctg ttg ccc aaa act tgg aca gag atg gat atc      138
   Ser Ser Lys Pro Ser Leu Leu Pro Lys Thr Trp Thr Glu Met Asp Ile
                   -95                 -90                 -85
   atg gaa cat gtt ggg caa aag ctc tgg aat cac aag ggg cgc agg gtt      186
   Met Glu His Val Gly Gln Lys Leu Trp Asn His Lys Gly Arg Arg Val
               -80                 -75                 -70
   cta cga ctc cgc ttc gtg tgt cag cag cca aga ggt agt gag gtt ctt      234
   Leu Arg Leu Arg Phe Val Cys Gln Gln Pro Arg Gly Ser Glu Val Leu
           -65                 -60                 -55
   gag ttc tgg tgg cat ggc act tca tca ttg gac act gtc ttc ttg tta      282
   Glu Phe Trp Trp His Gly Thr Ser Ser Leu Asp Thr Val Phe Leu Leu
       -50                 -45                 -40
   ctg tat ttc aat gac act cag agt gtt cag aag acc aaa cct ctc cct      330
   Leu Tyr Phe Asn Asp Thr Gln Ser Val Gln Lys Thr Lys Pro Leu Pro
   -35                 -30                 -25                 -20
   aaa ggc ctg aaa gag ttt aca gaa aaa gac cct tct ctt ctc ttg agg      378
   Lys Gly Leu Lys Glu Phe Thr Glu Lys Asp Pro Ser Leu Leu Leu Arg
                   -15                 -10                 -5
   agg gct cgt caa gca ggc agt att gca tcg gaa gtt cct ggc ccc tcc      426
   Arg Ala Arg Gln Ala Gly Ser Ile Ala Ser Glu Val Pro Gly Pro Ser
           -1  1               5                   10
   agg gag cat gat ggg cct gaa agt aac cag tgt tcc ctc cac cct ttt      474
   Arg Glu His Asp Gly Pro Glu Ser Asn Gln Cys Ser Leu His Pro Phe
       15                  20                  25
   caa gac agc ttc cag cag ctg ggc tgg gat cac tgg atc att gct ccc      522
   Gln Asp Ser Phe Gln Gln Leu Gly Trp Asp His Trp Ile Ile Ala Pro
   30                  35                  40                  45
   cat ctc tat acc cca aac tac tgt aag gga gta tgt cct cgg gta cta      570
   His Leu Tyr Thr Pro Asn Tyr Cys Lys Gly Val Cys Pro Arg Val Leu
                   50                  55                  60
   cac tat ggt ctc aat tct ccc aat cat gcc atc atc cag aac ctt gtc      618
   His Tyr Gly Leu Asn Ser Pro Asn His Ala Ile Ile Gln Asn Leu Val
               65                  70                  75
   agt gag ctg gtg gat cag aat gtc cct cag cct tcc tgt gtc cct tat      666
   Ser Glu Leu Val Asp Gln Asn Val Pro Gln Pro Ser Cys Val Pro Tyr
           80                  85                  90
   aag tat gtt ccc att agc atc ctt ctg att gag gca aat ggg agt atc      714
   Lys Tyr Val Pro Ile Ser Ile Leu Leu Ile Glu Ala Asn Gly Ser Ile
       95                  100                 105
   ttg tac aag gag tat gag ggt atg att gcc cag tcc tgc aca tgc agg      762
   Leu Tyr Lys Glu Tyr Glu Gly Met Ile Ala Gln Ser Cys Thr Cys Arg
   110                 115                 120                 125
   tgacggcaaa ggtgca                                                    778
<210> 2
   <211> 254
   <212> PRT
   <213> Ovis aries
<220>
   <221> misc_feature
   <222> (376)..(387)
   <223> furin protease sequence
<220>
   <221> misc_feature
   <222> (763)..(765)
   <223> stop codon
<400> 2
   Leu His Leu Thr His  Ser His Leu Ser Cys  His Val Glu Pro Trp
                   -125                 -120                 -115
   Val Gln Lys Ser Pro  Thr Asn His Phe Pro  Ser Ser Gly Arg Gly
                   -110                 -105                 -100
   Ser Ser Lys Pro Ser Leu Leu Pro Lys Thr Trp Thr Glu Met Asp Ile
                   -95                 -90                 -85
   Met Glu His Val Gly Gln Lys Leu Trp Asn His Lys Gly Arg Arg Val
               -80                 -75                 -70
   Leu Arg Leu Arg Phe Val Cys Gln Gln Pro Arg Gly Ser Glu Val Leu
           -65                 -60                 -55
   Glu Phe Trp Trp His Gly Thr Ser Ser Leu Asp Thr Val Phe Leu Leu
       -50                 -45                 -40
   Leu Tyr Phe Asn Asp Thr Gln Ser Val Gln Lys Thr Lys Pro Leu Pro
   -35                 -30                 -25                 -20
   Lys Gly Leu Lys Glu Phe Thr Glu Lys Asp Pro Ser Leu Leu Leu Arg
                   -15                 -10                 -5
   Arg Ala Arg Gln Ala Gly Ser Ile Ala Ser Glu Val Pro Gly Pro Ser
           -1  1               5                   10
   Arg Glu His Asp Gly Pro Glu Ser Asn Gln Cys Ser Leu His Pro Phe
       15                  20                  25
   Gln Asp Ser Phe Gln Gln Leu Gly Trp Asp His Trp Ile Ile Ala Pro
   30 35 40 45
   His Leu Tyr Thr Pro Asn Tyr Cys Lys Gly Val Cys Pro Arg Val Leu
   50 55 60
   His Tyr Gly Leu Asn Ser Pro Asn His Ala Ile Ile Gln Asn Leu Val
   65 70 75
   Ser Glu Leu Val Asp Gln Asn Val Pro Gln Pro Ser Cys Val Pro Tyr
   80 85 90
   Lys Tyr Val Pro Ile Ser Ile Leu Leu Ile Glu Ala Asn Gly Ser Ile
   95 100 105
   Leu Tyr Lys Glu Tyr Glu Gly Met Ile Ala Gln Ser Cys Thr Cys Arg
   110 115 120 125
<210> 3
   <211> 778
   <212> DNA
   <213> Ovis aries
<220>
   <221> CDS
   <222> (1)..(453)
<220> .
   <221> mat_peptide
   <222> (388)..()
<220>
   <221> mutation
   <222> (454)..(454)
   <223> Hanna nucleotide t but wildtype nucleotide c, Hanna codon tag bu t wildtype codon ca
<220>
   <221> misc_feature
   <222> (376)..(387)
   <223> furin protease sequence
<220>
   <221> misc_feature
   <222> (454)..(456)
   <223> premature stop codon
<400> 3
   ctt cac cta act cat tcc cac ctc tcc tgc cat gtg gag ccc tgg 45
   Leu His Leu Thr His Ser His Leu Ser Cys His Val Glu Pro Trp
                           -125                -120                -115
   gtc cag aaa agc cca  acc aat cac ttt cct  tct tca gga aga ggc       90
   Val Gln Lys Ser Pro  Thr Asn His Phe Pro  Ser Ser Gly Arg Gly
                   -110                -105                -100
   tcc tca aag cct tcc ctg ttg ccc aaa act tgg aca gag atg gat atc     138
   Ser Ser Lys Pro Ser Leu Leu Pro Lys Thr Trp Thr Glu Met Asp Ile
                   -95                 -90                 -85
   atg gaa cat gtt ggg caa aag ctc tgg aat cac aag ggg cgc agg gtt     186
   Met Glu His Val Gly Gln Lys Leu Trp Asn His Lys Gly Arg Arg Val
               -80                 -75                 -70
   cta cga ctc cgc ttc gtg tgt cag cag cca aga ggt agt gag gtt ctt     234
   Leu Arg Leu Arg Phe Val Cys Gln Gln Pro Arg Gly Ser Glu Val Leu
           -65                 -60                 -55
   gag ttc tgg tgg cat ggc act tca tca ttg gac act gtc ttc ttg tta     282
   Glu Phe Trp Trp His Gly Thr Ser Ser Leu Asp Thr Val Phe Leu Leu
       -50                 -45                 -40
   ctg tat ttc aat gac act cag agt gtt cag aag acc aaa cct ctc cct     330
   Leu Tyr Phe Asn Asp Thr Gln Ser Val Gln Lys Thr Lys Pro Leu Pro
   -35                 -30                 -25                 -20
   aaa ggc ctg aaa gag ttt aca gaa aaa gac cct tct ctt ctc ttg agg     378
   Lys Gly Leu Lys Glu Phe Thr Glu Lys Asp Pro Ser Leu Leu Leu Arg
                   -15                 -10                 -5
   agg gct cgt caa gca ggc agt att gca tcg gaa gtt cct ggc ccc tcc     426
   Arg Ala Arg Gln Ala Gly Ser Ile Ala Ser Glu Val Pro Gly Pro Ser
           -1  1               5                   10
   agg gag cat gat ggg cct gaa agt aac tagtgttccc tccacccttt           473
   Arg Glu His Asp Gly Pro Glu Ser Asn
       15                  20
   tcaagtcagc ttccagcagc tgggctggga tcactggatc attgctcccc atctctatac   533
   cccaaactac tgtaagggag tatgtcctcg ggtactacac tatggtctca attctcccaa   593
   tcatgccatc atccagaacc ttgtcagtga gctggtggat cagaatgtcc ctcagccttc   653
   ctgtgtccct tataagtatg ttcccattag catccttctg attgaggcaa atgggagtat   713
   cttgtacaag gagtatgagg gtatgattgc ccagtcctgc acatgcaggt gacggcaaag   773
   gtgca                                                               778
<210> 4
   <211> 151
   <212> PRT
   <213> Ovis aries
<220>
   <221> misc_feature
   <222> (376)..(387)
   <223> furin protease sequence
<220>
   <221> misc_feature
   <222> (454)..(456)
   <223> premature stop codon
<400> 4
   Leu His Leu Thr His  Ser His Leu Ser Cys  His Val Glu Pro Trp
                   -125                 -120                 -115
   Val Gln Lys Ser Pro  Thr Asn His Phe Pro  Ser Ser Gly Arg Gly
                   -110                 -105                 -100
   Ser Ser Lys Pro Ser Leu Leu Pro Lys Thr Trp Thr Glu Met Asp Ile
                   -95                 -90                 -85
   Met Glu His Val Gly Gln Lys Leu Trp Asn His Lys Gly Arg Arg Val
               -80                 -75                 -70
   Leu Arg Leu Arg Phe Val Cys Gln Gln Pro Arg Gly Ser Glu Val Leu
           -65                 -60                 -55
   Glu Phe Trp Trp His Gly Thr Ser Ser Leu Asp Thr Val Phe Leu Leu
       -50                 -45                 -40
   Leu Tyr Phe Asn Asp Thr Gln Ser Val Gln Lys Thr Lys Pro Leu Pro
   -35                 -30                 -25                 -20
   Lys Gly Leu Lys Glu Phe Thr Glu Lys Asp Pro Ser Leu Leu Leu Arg
                   -15                 -10                 -5
   Arg Ala Arg Gln Ala Gly Ser Ile Ala Ser Glu Val Pro Gly Pro Ser
           -1  1               5                   10
   Arg Glu His Asp Gly Pro Glu Ser Asn
       15                  20
<210> 5
   <211> 391
   <212> DNA
   <213> Ovis aries
<220>
   <221> CDS
   <222> (1)..(375)
<220>
   <221> mutation
   <222> (92)..(92)
<220>
   <221> misc_feature
   <222> (376)..(378)
   <223> stop codon
<400> 5
   caa gca ggc agt att gca tcg gaa gtt cct ggc ccc tcc agg gag cat        48
   Gln Ala Gly Ser Ile Ala Ser Glu Val Pro Gly Pro Ser Arg Glu His
   1               5                   10                  15
   gat ggg cct gaa agt aac cag tgt tcc ctc cac cct ttt caa gac agc        96
   Asp Gly Pro Glu Ser Asn Gln Cys Ser Leu His Pro Phe Gln Asp Ser
               20                  25                  30
   ttc cag cag ctg ggc tgg gat cac tgg atc att gct ccc cat ctc tat       144
   Phe Gln Gln Leu Gly Trp Asp His Trp Ile Ile Ala Pro His Leu Tyr
           35                  40                  45
   acc cca aac tac tgt aag gga gta tgt cct cgg gta cta cac tat ggt       192
   Thr Pro Asn Tyr Cys Lys Gly Val Cys Pro Arg Val Leu His Tyr Gly
       50                  55                  60
   ctc aat tct ccc aat cat gcc atc atc cag aac ctt gtc agt gag ctg       240
   Leu Asn Ser Pro Asn His Ala Ile Ile Gln Asn Leu Val Ser Glu Leu
   65                  70                  75                  80
   gtg gat cag aat gtc cct cag cct tcc tgt gtc cct tat aag tat gtt       288
   Val Asp Gln Asn Val Pro Gln Pro Ser Cys Val Pro Tyr Lys Tyr Val
                   85                  90                  95
   ccc att agc atc ctt ctg att gag gca aat ggg agt atc ttg tac aag       336
   Pro Ile Ser Ile Leu Leu Ile Glu Ala Asn Gly Ser Ile Leu Tyr Lys
               100                 105                 110
   gag tat gag ggt atg att gcc cag tcc tgc aca tgc agg tgacggcaaa        385
   Glu Tyr Glu Gly Met Ile Ala Gln Ser Cys Thr Cys Arg
           115                 120                 125
   ggtgca                                                                391
<210> 6
   <211> 125
   <212> PRT
   <213> Ovis aries
<220>
   <221> misc_feature
   <222> (376)..(378)
   <223> stop codon
<400> 6
   Gln Ala Gly Ser Ile Ala Ser Glu Val Pro Gly Pro Ser Arg Glu His
   1               5                   10                  15
   Asp Gly Pro Glu Ser Asn Gln Cys Ser Leu His Pro Phe Gln Asp Ser
               20                  25                  30
   Phe Gln Gln Leu Gly Trp Asp His Trp Ile Ile Ala Pro His Leu Tyr
           35                  40                  45
   Thr Pro Asn Tyr Cys Lys Gly Val Cys Pro Arg Val Leu His Tyr Gly
       50                  55                  60
   Leu Asn Ser Pro Asn His Ala Ile Ile Gln Asn Leu Val Ser Glu Leu
   65                  70                  75                  80
   Val Asp Gln Asn Val Pro Gln Pro Ser Cys Val Pro Tyr Lys Tyr Val
                   85                  90                  95
   Pro Ile Ser Ile Leu Leu Ile Glu Ala Asn Gly Ser Ile Leu Tyr Lys
               100                 105                 110
   Glu Tyr Glu Gly Met Ile Ala Gln Ser Cys Thr Cys Arg
           115                 120                 125
<210> 7
   <211> 391
   <212> DNA
   <213> Ovis aries
<220>
   <221> CDS
   <222> (1)..(66)
<220>
   <221> mutation
   <222> (67)..(67)
<220>
   <221> misc_feature
   <222> (67)..(69)
   <223> premature stop codon
<400> 7
   caa gca ggc agt att gca tcg gaa gtt cct ggc ccc tcc agg gag cat        48
   Gln Ala Gly Ser Ile Ala Ser Glu Val Pro Gly Pro Ser Arg Glu His
   1               5                   10                  15
   gat ggg cct gaa agt aac tagtgttccc tccacccttt tcaagtcagc                96
   Asp Gly Pro Glu Ser Asn
               20
   ttccagcagc tgggctggga tcactggatc attgctcccc atctctatac cccaaactac     156
   tgtaagggag tatgtcctcg ggtactacac tatggtctca attctcccaa tcatgccatc    216
   atccagaacc ttgtcagtga gctggtggat cagaatgtcc ctcagccttc ctgtgtccct    276
   tataagtatg ttcccattag catccttctg attgaggcaa atgggagtat cttgtacaag    336
   gagtatgagg gtatgattgc ccagtcctgc acatgcaggt gacggcaaag gtgca         391
<210> 8
   <211> 22
   <212> PRT
   <213> Ovis aries
<220>
   <221> misc_feature
   <222> (67)..(69)
   <223> premature stop codon
<400> 8
   Gln Ala Gly Ser Ile Ala Ser Glu Val Pro Gly Pro Ser Arg Glu His
   1               5                   10                  15
   Asp Gly Pro Glu Ser Asn
               20
<210> 9
   <211> 778
   <212> DNA
   <213> Ovis aries
<220>
   <221> CDS
   <222> (1)..(762)
<220>
   <221> misc_feature
   <222> (376)..(387)
   <223> furin protease sequence
<220>
   <221> mat_peptide
   <222> (388) .. ()
<220>
   <221> misc_feature
   <222> (479)..(479)
   <223> position of Inverdale mutation
<220>
   <221> misc_feature
   <222> (454)..(454)
   <223> position of Hanna mutation
<220>
   <221> misc_feature
   <222> (763)..(765)
   <223> stop codon
<400> 9
   ctt cac cta act cat  tcc cac ctc tcc tgc  cat gtg gag ccc tgg       45
   Leu His Leu Thr His  Ser His Leu Ser Cys  His Val Glu Pro Trp
                   -125                 -120                 -115
   gtc cag aaa agc cca  acc aat cac ttt cct  tct tca gga aga ggc       90
   Val Gln Lys Ser Pro  Thr Asn His Phe Pro  Ser Ser Gly Arg Gly
                   -110                 -105                 -100
   tcc tca aag cct tcc ctg ttg ccc aaa act tgg aca gag atg gat atc    138
   Ser Ser Lys Pro Ser Leu Leu Pro Lys Thr Trp Thr Glu Met Asp Ile
                   -95                 -90                 -85
   atg gaa cat gtt ggg caa aag ctc tgg aat cac aag ggg cgc agg gtt    186
   Met Glu His Val Gly Gln Lys Leu Trp Asn His Lys Gly Arg Arg Val
               -80                 -75                 -70
   cta cga ctc cgc ttc gtg tgt cag cag cca aga ggt agt gag gtt ctt    234
   Leu Arg Leu Arg Phe Val Cys Gln Gln Pro Arg Gly Ser Glu Val Leu
           -65                 -60                 -55
   gag ttc tgg tgg cat ggc act tca tca ttg gac act gtc ttc ttg tta    282
   Glu Phe Trp Trp His Gly Thr Ser Ser Leu Asp Thr Val Phe Leu Leu
       -50                 -45                 -40
   ctg tat ttc aat gac act cag agt gtt cag aag acc aaa cct ctc cct    330
   Leu Tyr Phe Asn Asp Thr Gln Ser Val Gln Lys Thr Lys Pro Leu Pro
   -35                 -30                 -25                 -20
   aaa ggc ctg aaa gag ttt aca gaa aaa gac cct tct ctt ctc ttg agg    378
   Lys Gly Leu Lys Glu Phe Thr Glu Lys Asp Pro Ser Leu Leu Leu Arg
                   -15                 -10                 -5
   agg gct cgt caa gca ggc agt att gca tcg gaa gtt cct ggc ccc tcc    426
   Arg Ala Arg Gln Ala Gly Ser Ile Ala Ser Glu Val Pro Gly Pro Ser
           -1  1               5                   10
   agg gag cat gat ggg cct gaa agt aac cag tgt tcc ctc cac cct ttt    474
   Arg Glu His Asp Gly Pro Glu Ser Asn Gln Cys Ser Leu His Pro Phe
       15                  20                  25
   caa gtc agc ttc cag cag ctg ggc tgg gat cac tgg atc att gct ccc    522
   Gln Val Ser Phe Gln Gln Leu Gly Trp Asp His Trp Ile Ile Ala Pro
   30                  35                  40                  45
   cat ctc tat acc cca aac tac tgt aag gga gta tgt cct cgg gta cta    570
   His Leu Tyr Thr Pro Asn Tyr Cys Lys Gly Val Cys Pro Arg Val Leu
                   50                  55                  60
   cac tat ggt ctc aat tct ccc aat cat gcc atc atc cag aac ctt gtc    618
   His Tyr Gly Leu Asn Ser Pro Asn His Ala Ile Ile Gln Asn Leu Val
                       65                            70                            75
   agt gag ctg gtg gat cag aat gtc cct cag cct tcc tgt gtc cct tat      666
   Ser Glu Leu Val Asp Gln Asn Val Pro Gln Pro Ser Cys Val Pro Tyr
           80                  85                  90
   aag tat gtt ccc att agc atc ctt ctg att gag gca aat ggg agt atc      714
   Lys Tyr Val Pro Ile Ser Ile Leu Leu Ile Glu Ala Asn Gly Ser Ile
       95                  100                 105
   ttg tac aag gag tat gag ggt atg att gcc cag tcc tgc aca tgc agg      762
   Leu Tyr Lys Glu Tyr Glu Gly Met Ile Ala Gln Ser Cys Thr Cys Arg
   110                 115                 120                 125
   tgacggcaaa ggtgca                                                    778
<210> 10
   <211> 254
   <212> PRT
   <213> Ovis aries
<220>
   <221> misc_feature
   <222> (376)..(387)
   <223> furin protease sequence
<220>
   <221> misc_feature
   <222> (479)..(479)
   <223> position of Inverdale mutation
<220>
   <221> misc_feature
   <222> (454)..(454)
   <223> position of Hanna mutation
<220>
   <221> misc_feature
   <222> (763)..(765)
   <223> stop codon
<400> 10
   Leu His Leu Thr His Ser His Leu Ser Cys His Val Glu Pro Trp
                   -125                -120                -115
   Val Gln Lys Ser Pro Thr Asn His Phe Pro Ser Ser Gly Arg Gly
                   -110                -105                -100
   Ser Ser Lys Pro Ser Leu Leu Pro Lys Thr Trp Thr Glu Met Asp Ile
                   -95                 -90                 -85
   Met Glu His Val Gly Gln Lys Leu Trp Asn His Lys Gly Arg Arg Val
               -80                 -75                 -70
   Leu Arg Leu Arg Phe Val Cys Gln Gln Pro Arg Gly Ser Glu Val Leu
           -65                 -60                 -55
   Glu Phe Trp Trp His Gly Thr Ser Ser Leu Asp Thr Val Phe Leu Leu
       -50                 -45                 -40
   Leu Tyr Phe Asn Asp Thr Gln Ser Val Gln Lys Thr Lys Pro Leu Pro
   -35                 -30                 -25                 -20
   Lys Gly Leu Lys Glu Phe Thr Glu Lys Asp Pro Ser Leu Leu Leu Arg
                   -15                 -10                 -5
   Arg Ala Arg Gln Ala Gly Ser Ile Ala Ser Glu Val Pro Gly Pro Ser
           -1  1               5                   10
   Arg Glu His Asp Gly Pro Glu Ser Asn Gln Cys Ser Leu His Pro Phe
       15                  20                  25
   Gln Val Ser Phe Gln Gln Leu Gly Trp Asp His Trp Ile Ile Ala Pro
   30                  35                  40                  45
   His Leu Tyr Thr Pro Asn Tyr Cys Lys Gly Val Cys Pro Arg Val Leu
                   50                  55                  60
   His Tyr Gly Leu Asn Ser Pro Asn His Ala Ile Ile Gln Asn Leu Val
               65                  70                  75
   Ser Glu Leu Val Asp Gln Asn Val Pro Gln Pro Ser Cys Val Pro Tyr
           80                  85                  90
   Lys Tyr Val Pro Ile Ser Ile Leu Leu Ile Glu Ala Asn Gly Ser Ile
       95                  100                 105
   Leu Tyr Lys Glu Tyr Glu Gly Met Ile Ala Gln Ser Cys Thr Cys Arg
   110                 115                 120                 125
<210> 11
   <211> 16
   <212> PRT
   <213> Ovis aries
<400> 11
   Ser Glu Val Pro Gly Pro Ser Arg Glu His Asp Gly Pro Glu Ser Cys
   1               5                   10                  15
<210> 12
   <211> 206
   <212> DNA
   <213> Ovis aries
<220>
   <221> CDS
   <222> (1)..(204) .
   <223> subset of Inverdale GDF9B nucleotide sequence around the mutation
<220>
   <221> mutation
   <222> (86)..(86)
   <223> position of Inverdale mutation
<400> 12
   ggc agt att gca tcg gaa gtt cct ggc ccc tcc agg gag cat gat ggg      48
   Gly Ser Ile Ala Ser Glu Val Pro Gly Pro Ser Arg Glu His Asp Gly
   1               5                   10                  15
   cct gaa agt aac cag tgt tcc ctc cac cct ttt caa gac agc ttc cag      96
   Pro Glu Ser Asn Gln Cys Ser Leu His Pro Phe Gln Asp Ser Phe Gln
               20                  25                  30
   cag ctg ggc tgg gat cac tgg atc att gct ccc cat ctc tat acc cca      144
   Gln Leu Gly Trp Asp His Trp Ile Ile Ala Pro His Leu Tyr Thr Pro
           35                  40                  45
   aac tac tgt aag gga gta tgt cct cgg gta cta cac tat ggt ctc aat      192
   Asn Tyr Cys Lys Gly Val Cys Pro Arg Val Leu His Tyr Gly Leu Asn
       50                  55                  60
   tct ccc aat cat gc                                                   206
   Ser Pro Asn His
   65
<210> 13
   <211> 68
   <212> PRT
   <213> Ovis aries
<400> 13
   Gly Ser Ile Ala Ser Glu Val Pro Gly Pro Ser Arg Glu His Asp Gly
   1               5                   10                  15
   Pro Glu Ser Asn Gln Cys Ser Leu His Pro Phe Gln Asp Ser Phe Gln
               20                  25                  30
   Gln Leu Gly Trp Asp His Trp Ile Ile Ala Pro His Leu Tyr Thr Pro
           35                  40                  45
   Asn Tyr Cys Lys Gly Val Cys Pro Arg Val Leu His Tyr Gly Leu Asn
   50 55 60
   Ser Pro Asn His
   65
<210> 14
   <211> 206
   <212> DNA
   <213> Ovis aries
<220>
   <221> CDS
   <222> (1) .. (60)
   <223> subset of Hanna GDF9B nucleotide sequence around the mutation
<220>
   <221> mutation
   <222> (61)..(61)
   <223> position of Hanna mutation
<400> 14
   ggc agt att gca tcg gaa gtt cct ggc ccc tcc agg gag cat gat ggg        48
   Gly Ser Ile Ala Ser Glu Val Pro Gly Pro Ser Arg Glu His Asp Gly
   1               5                   10                  15
   cct gaa agt aac tagtgttccc tccacccttt tcaagtcagc ttccagcagc           100
   Pro Glu Ser Asn
               20
   tgggctggga tcactggatc attgctcccc atctctatac cccaaactac tgtaagggag      160
   tatgtcctcg ggtactacac tatggtctca attctcccaa tcatgc                    206
<210> 15
   <211> 20
   <212> PRT
   <213> Ovis aries
<400> 15
   Gly Ser Ile Ala Ser Glu Val Pro Gly Pro Ser Arg Glu His Asp Gly
   1               5                   10                  15
   Pro Glu Ser Asn
               20
<210> 16
   <211> 1519
   <212> DNA
   <213> Trichosurus vulpecula
<220>
   <221> Intron
   <222> (1)..(712)
<220>
   <221> CDS
   <222> (713)..(1519)
<220>
   <221> mat_peptide
   <222> (1178)..()
<220>
   <221> exon
   <222> (713)..(1519)
<220>
   <221> misc_feature
   <222> (1166)..(1177)
   <223> furin protease sequence
<400> 16
   tttgtttatc tatgtcccaa atattttttt ctccttttgg agcaaatgca aaggaaaggg      60
   acttagctgg tgaagaatca gggtaggttg gaacacccac taaggaaagt gaaactatag     120
   aaagagcact aggtctggac ctgggttaga atcctgtctt tgccacatct tagccgtgta     180
   accttaggca agtggcttaa cttctctggg ccttcatttt atcttctgta aaatgagaca     240
   tttccaactg tggtctccat gcatttgcat tagctgttcc ctgtgcctgg aatgccctcc     300
   ctcctttgtg tctcagaatc cttaatcttt cttctatctt cttttcttct cttcccctac     360
   ttcccagtta ctactgctct ctccctcctc aaatcacatt atgctgttct tacccattcg     420
   cacattatcg gattccaatc ctgctctctg cacggccccc cacccccggt agaacatgag     480
   cttcttgaag gccaggcttg tttttcctct ctatggtgcc tgacatatac aggagcttaa     540
   taaacacttg ttgaccagat agtgtggagc tggctttgag ggggaagtga acctccccct     600
   aattggtcat ttatgatgcc aagggagaag gcctaacaga actcttctct tggtcaggtg     660
   ggttgggaag ctgacattct gttcttctta ccaccttcat tttctctgtg ca ggt         715
                                                             Gly
                                                             -155
   ccc tgg tat gtg caa acc cta gac ttc cca ctt cgg cca aac cgt           760
   Pro Trp Tyr Val Gln Thr Leu Asp Phe Pro Leu Arg Pro Asn Arg
                   -150                -145                -140
   gac atg gac cat ctg gtg agg gcc gct gtt gct tac cgc cct cgc           805
   Asp Met Asp His Leu Val Arg Ala Ala Val Ala Tyr Arg Pro Arg
                   -135                -130                -125
   ctc cgc cta tct cac tcc cat ctt tcc tgc cat gtg gaa ccc tgg           850
   Leu Arg Leu Ser His Ser His Leu Ser Cys His Val Glu Pro Trp
                   -120                -115                -110
   gcc cac aag agc acc  atc ctc ctg gga gga  ggc tcc cca ggc ttt gct      898
   Ala His Lys Ser Thr  Ile Leu Leu Gly Gly  Gly Ser Pro Gly Phe Ala
                   -105                 -100                 -95
   ttg ccg gag gcc tgg gca gaa atg gat ctc acc aat tac att cag cag        946
   Leu Pro Glu Ala Trp Ala Glu Met Asp Leu Thr Asn Tyr Ile Gln Gln
               -90                 -85                 -80
   caa gtt cag cct caa aag ggg agg aga gtc ctt cac atc caa gtc agg        994
   Gln Val Gln Pro Gln Lys Gly Arg Arg Val Leu His Ile Gln Val Arg
           -75                 -70                 -65
   tgt cag cag caa gaa agg aca gag att ggc ctt ggg tgg agg cag gcc       1042
   Cys Gln Gln Gln Glu Arg Thr Glu Ile Gly Leu Gly Trp Arg Gln Ala
       -60                 -55                 -50
   ttg gct act gac act gct ttc ctg gtt ctg tat ttc aac aat acc ttt       1090
   Leu Ala Thr Asp Thr Ala Phe Leu Val Leu Tyr Phe Asn Asn Thr Phe
   -45                 -40                 -35                 -30
   aaa agt gtg cca cga atg gag ctg cca gaa ctt ctg gtg ggg gac cct       1138
   Lys Ser Val Pro Arg Met Glu Leu Pro Glu Leu Leu Val Gly Asp Pro
                   -25                 -20                 -15
   gag gga gcc gat ctc ctt tcc ctg gcc cgg cga gtc cgt cag gtg ggc       1186
   Glu Gly Ala Asp Leu Leu Ser Leu Ala Arg Arg Val Arg Gln Val Gly
               -10                 -5                  -1   1
   cct gta agg tct gaa gca cct ggc cag tca ctg gag cag aca cag tgt       1234
   Pro Val Arg Ser Glu Ala Pro Gly Gln Ser Leu Glu Gln Thr Gln Cys
       5                   10                  15
   tct ctc cat cct ttc cag gtt agc ttc cac cag ctg ggc tgg gag aac       1282
   Ser Leu His Pro Phe Gln Val Ser Phe His Gln Leu Gly Trp Glu Asn
   20                  25                  30                  35
   tgg atc att gcc ccc cat ctg tac agc cca aac tac tgc aag ggg gcc       1330
   Trp Ile Ile Ala Pro His Leu Tyr Ser Pro Asn Tyr Cys Lys Gly Ala
                   40                  45                  50
   tgt cca cgg gtg cta cac tct ggc ctc cga tca ccc aac cat gcc atc       1378
   Cys Pro Arg Val Leu His Ser Gly Leu Arg Ser Pro Asn His Ala Ile
               55                  60                  65
   att cag aac ctt atc aac gag ctg gtg gat aga agc atc ccc cga ccc       1426
   Ile Gln Asn Leu Ile Asn Glu Leu Val Asp Arg Ser Ile Pro Arg Pro
           70                  75                  80
   tca tgc gtc cct tac aag tac atg ccc att agt gtc ctg ctg att gag       1474
   Ser Cys Val Pro Tyr Lys Tyr Met Pro Ile Ser Val Leu Leu Ile Glu
       85                  90                  95
   gcc agt ggc agc atc ctg tac aaa gaa tat gag gac atg att gcc           1519
   Ala Ser Gly Ser Ile Leu Tyr Lys Glu Tyr Glu Asp Met Ile Ala
   100                 105                 110
<210> 17
   <211> 269
   <212> PRT
   <213> Trichosurus vulpecula
<220>
   <221> misc_feature
   <222> (1166)..(1177)
   <223> furin protease sequence
<400> 17
   Gly  Pro Trp Tyr Val Gln  Thr Leu Asp Phe Pro  Leu Arg Pro Asn
   -155                 -150                 -145
   Arg  Asp Met Asp His Leu  Val Arg Ala Ala Val  Ala Tyr Arg Pro
   -140                 -135                 -130
   Arg  Leu Arg Leu Ser His  Ser His Leu Ser Cys  His Val Glu Pro
   -125                 -120                 -115
   Trp  Ala His Lys Ser Thr  Ile Leu Leu Gly Gly  Gly Ser Pro Gly
   -110                 -105                 -100
   Phe Ala Leu Pro Glu Ala Trp Ala Glu Met Asp Leu Thr Asn Tyr Ile
   -95                 -90                 -85                 -80
   Gln Gln Gln Val Gln Pro Gln Lys Gly Arg Arg Val Leu His Ile Gln
                   -75                 -70                 -65
   Val Arg Cys Gln Gln Gln Glu Arg Thr Glu Ile Gly Leu Gly Trp Arg
               -60                 -55                 -50
   Gln Ala Leu Ala Thr Asp Thr Ala Phe Leu Val Leu Tyr Phe Asn Asn
           -45                 -40                 -35
   Thr Phe Lys Ser Val Pro Arg Met Glu Leu Pro Glu Leu Leu Val Gly
       -30                 -25                 -20
   Asp Pro Glu Gly Ala Asp Leu Leu Ser Leu Ala Arg Arg Val Arg Gln
   -15                 -10                 -5                  -1  1
   Val Gly Pro Val Arg Ser Glu Ala Pro Gly Gln Ser Leu Glu Gln Thr
               5                   10                  15
   Gln Cys Ser Leu His Pro Phe Gln Val Ser Phe His Gln Leu Gly Trp
           20                  25                  30
   Glu Asn Trp Ile Ile Ala Pro His Leu Tyr Ser Pro Asn Tyr Cys Lys
   35 40 45
   Gly Ala Cys Pro Arg Val Leu His Ser Gly Leu Arg Ser Pro Asn His
   50 55 60 65
   Ala Ile Ile Gln Asn Leu Ile Asn Glu Leu Val Asp Arg Ser Ile Pro
   70 75 80
   Arg Pro Ser Cys Val Pro Tyr Lys Tyr Met Pro Ile Ser Val Leu Leu
   85 90 95
   Ile Glu Ala Ser Gly Ser Ile Leu Tyr Lys Glu Tyr Glu Asp Met Ile
   100 105 110
   Ala